# EUROPEAN PATENT SPECIFICATION

(11) **EP 2 340 026 B1**
(45) Date of publication and mention of the grant of the patent: **06.12.2017**
(21) Application number: 09815639.1
(22) Date of filing: 29.09.2009
(51) Int. Cl.: A61K 35/14, A61P 3/10, A61P 25/02

(54) **A DEPROTEINISED CALF BLOOD PREPARATION FOR USE IN PREVENTION OR TREATMENT OF DIABETIC PERIPHERAL POLYNEUROPATHY**
DEPROTEINISIERTE KALBSBLUTZUBEREITUNG ZUR VERWENDUNG FÜR DIE VORBEUGUNG ODER BEHANDLUNG VON PERIPHERER DIABETES-POLYNEUROPATIE
PRÉPARATION DE SANG DE VEAU SANS PROTÉINE POUR L'UTILISATION DANS LA PRÉVENTION OU LE TRAITEMENT DE POLYNEUROPATHIE PÉRIPHÉRIQUE DIABÉTIQUE

(30) Priority: 29.09.2008 EP 08165446; 20.05.2009 EP 09160858
(43) Date of publication of application: 06.07.2011
(73) Proprietor: Takeda Austria GmbH, 4020 Linz (AT)
(72) Inventor: MOVSESYAN, LUSINE, DK-4000 Roskilde (DK)
(74) Representative: Plougmann Vingtoft a/s
(86) International application number: PCT/DK2009/050251
(87) International publication number: WO 2010/034315

(56) References cited:
- DE-B- 1 076 888
- DATABASE WPI Week 200674 Thomson Scientific, London, GB; AN 2006-715518 XP002533168 & RU 2 282 453 C2 (BOYKO I N) 27 August 2006 (2006-08-27) cited in the application
- JANSEN, W. AND BECK, E: "Behandlung der diabetischen Polyneuropathie - eine kontrollierte Doppelblindstudie" DIE MEDIZINISCHE WELT, vol. 38, 1987, pages 838-841, XP008107284 cited in the application
- DATABASE WPI Week 200739 Thomson Scientific, London, GB; AN 2007-404963 XP002533169 & CN 1 895 274 A (AOHONG PHARM CO LTD) 17 January 2007 (2007-01-17) cited in the application
- DATABASE MEDLINE [Online] US NATIONAL LIBRARY OF MEDICINE (NLM), BETHESDA, MD, US; 1997, SIMEONOV S ET AL: "Therapeutic efficacy of "Milgamma" in patients with painful diabetic neuropathy." XP002533167 Database accession no. NLM9575643 cited in the application & FOLIA MEDICA 1997, vol. 39, no. 4, 1997, pages 5-10, ISSN: 0204-8043
- ZIEGLER D ET AL: "Oral treatment with [alpha]-lipoic acid improves symptomatic diabetic polyneuropathy" DIABETES CARE 200611 US, vol. 29, no. 11, November 2006 (2006-11), pages 2365-2370, XP002533165 ISSN: 0149-5992 0149-5992 cited in the application
- ZIEGLER D: "Management of painful diabetic neuropathy: What is new or in the pipeline for 2007?" CURRENT DIABETES REPORTS 200712 GB, vol. 7, no. 6, December 2007 (2007-12), pages 409-415, XP008107258 ISSN: 1534-4827 cited in the application
- ZIEGLER ET AL.,: "Treatment of Symptomatic Polyneuropathy with Actovegin in Type 2 Diabetic Patients"[Online] XP002533166 INTERNET Retrieved from the Internet: URL:http://care.diabetesjournals.org/conte nt/early/2009/05/22/dc09-0545.abstract>

## Description

### Technical field of the invention

The present invention relates to a deproteinised calf blood preparation for use in prevention or treatment of diabetic peripheral polyneuropathy, wherein the deproteinised calf blood preparation is administered parenterally at least once and wherein the parenteral administration is followed by a period of enteral administration.

The preferred deproteinised calf blood preparation is the medicament Actovegin®.

### Background of the invention

Diabetic peripheral polyneuropathy (DPN) is the type of peripheral neuropathy associated with diabetes mellitus. Peripheral polyneuropathy (DPN) is a medical term used to define a deranged function and structure of peripheral motor, sensory, and autonomic neurons, involving either the entire neuron or selected levels. Peripheral polyneuropathy (DPN) mainly affects the feet and legs and leads to substantial morbidity; it is encountered in more than one third of diabetic patients and is associated with increased mortality in relation to its severity and complications, such as foot ulcers.

DNP is associated with excruciating neuropathic pain and foot ulcers leading to amputation (Boulton AJM et al., Diabetes Care 28, 956-962, 2005). Neuropathic pain may affect up to 26% of the diabetes population (Davies M et al., Diabetes Care 29: 1518-1522, 2006) and can exert a substantial impact on quality of life, particularly through the impairment of sleep and reduced enjoyment of life (Galer BS et al., Diabetes Res Clin Pract 47: 123-128, 2000). Several classes of analgesics are effective in the treatment of neuropathic pain, but no more than 40-60% of patients show adequate pain relief on monotherapy (Dworkin RH et al.,Pain 132: 237-251, 2007). Moreover, these drugs are frequently associated with central nervous system side effects and do not slow the progression of the underlying neuropathy (Boulton AJM et al., Diabetes Care 28, 956-962, 2005). Based on the pathogenetic mechanisms of DPN (Cameron NE et al., Diabetologia 44: 1973-1988, 2001), several therapeutic approaches have been developed (Boulton AJM et al., Diabetes Care 28, 956-962, 2005, Chalk C et al. Cochrane Database Syst Rev 4: CD004572, 2007, Ziegler D et al., Diabetologia 38: 1425-1433, 1995). These drugs have been designed to favourably influence the pathophysiology of the disorder, rather than simply relieving pain. However, despite apparent recent progress, the pharmacologic treatment of chronic symptomatic DPN remains a challenge for the physician (Dworkin RH et al.,Pain 132: 237-251, 2007).

Adequate epidemiological information about the incidence and natural history of peripheral neuropathy in diabetic patients is still lacking, although it is a common and clinically significant complication (Coppini DV et al. J. Clinical Neuros (2001), 8(6), 520-524). It is generally believed that keeping near-normoglycemia levels is the primary approach to prevent diabetic neuropathy (Ziegler D et al., Diabetes Care, Vol 22 (8), 1999, p 1296-1301). A 12 years prospective study on long term progression of diabetic peripheral neuropathy demonstrated that maintaining non-ideal long term glycaemic control lead to neuropathy in a about 20% of the patients correlating to a predisposed subset of patients (Coppini DV et al. J. Clinical Neuros (2001), 8(6), 520-524).

Actovegin® is a deproteinized hemoderivative produced from calf blood by ultrafiltration that contains low molecular weight compounds of up to 5000 Dalton. Oxygen absorption, oxygen utilization, and cellular energy metabolism is described as being affected by Actovegin® (Kuninaka T et al. J Cell Physiol 146: 148-155, 1991). Furthermore, Actovegin® is described as having insulin-like activity, such as stimulation of glucose transport, pyruvate dehydrogenase (PDH), and glucose oxidation (Obermaier-Kusser B et al., Biochem J 261: 699-705, 1989, Jacob S et al. Arzneimittelforschung 46: 269-272, 1996). Because of these properties, Actovegin® has previously been used for treatment of cerebral vascular and degenerative disorders (Kanowski S et al., Pharmacopsychiat 28: 125-133, 1995, Herrmann WM et al., Z Geriatrie 5: 46-55, 1992).

A study of 70 diabetic patients who received oral treatment with 600 mg of Actovegin® three times daily for 24 weeks found an optimal effect after 16 weeks (Jansen W. and Beck E, Die Medizinske Welt, Behandlung der diabetischen Polyneuropathie - eine kontrollierte Doppelblindstudie, medwelt 1986, 37:838-841, 1987).

Patent document RU 2 282 453 C2 (14.12.2004) discloses in addition to conventional therapy for NIDD dealing with introduction of manil-5, vitamin complex and a diet (N9), the intravenous injection of Actovegin®. Actovegin® is injected intravenously at the dosage of 400 mg twice daily for 10 days, then - intramuscularly per 200 mg twice daily for 10-15 days, then per 2 dragees 200 mg thrice 30 days. After 3 months of therapy, it is necessary to repeat the treatment using Actovegin® per 2 dragee 200 mg twice or thrice daily for 15 days. Such implementation enables to normalize carbohydrate metabolism and improve microcirculation in affected organs and tissues.

A variety of experimental studies have provided new insight into the putative pathogenesis of diabetic neuropathy. It has been suggested that nerve ischemia and hypoxia might play a paramount role in the pathogenesis of DPN. Reduced nerve blood flow in experimental DPN may be prevented and corrected by several disease-modifying drugs (Cameron NE et al., Diabetologia 44: 1973-1988, 2001). Several potential forms of treatment based on these pathogenetic considerations have been developed. Oxidative stress has been suggested to be implicated in diabetic polyneuropathy (DPN). Hence a study was conducted with the antioxidant α-lipoic acid comprising 3 weeks intravenous administration indicating that intravenous treatment with alpha-lipoic acid using a dose of 600 mg/day over 3 weeks is superior to placebo in reducing symptoms of diabetic peripheral neuropathy (Ziegler D et al., Diabetologia, Vol 38 (12), 1995, 1425-33).

However, these results could not be confirmed in a follow-up study on 509 patients which were randomly assigned to sequential treatment with 600 mg alpha-lipoic acid once daily intraveneously for 3 weeks, followed by 600 mg alpha-lipoic acid three times a day orally for 6 months, 600 mg alpha-lipoic acid once daily intraveneously for 3 weeks, followed by placebo three times a day orally for 6 months, and placebo once daily intraveneously for 3 weeks, followed by placebo three times a day orally for 6 months, Outcome measures included the Total Symptom Score (TSS) for neuropathic symptoms in the feet and the Neuropathy Impairment Score (NIS). Data analysis was based on the intention to treat (ITT). After 7 months, the changes in the TSS from baseline were not significantly different between the three groups studied. It was concluded by the authors that the treatment had no effect on neuropathic symptoms distinguishable from placebo (Ziegler D et al., Diabetes Care, Vol 22 (8), 1999, 1296-1301).

Hence, there is an urgent need for an effective treatment of diabetic neuropathy.

### Summary of the invention

An object of the present invention is to provide a treatment of diabetic neuropathy (DNP) which can significantly reduce the neuropathic symptoms as evidenced by the Total Symptom Score (TSS) and the risk of complications, such as foot ulcers, as evidenced by a decrease in the Vibration Perception Threshold (VPT), a predictor for future risk of foot ulceration.

This object is met by the present invention which provides a deproteinised calf blood preparation for use in prevention or treatment of diabetic peripheral polyneuropathy, wherein the deproteinised calf blood preparation is administered parenterally at least once and wherein the parenteral administration is followed by a period of enteral administration. Preferably, the deproteinised calf blood preparation is administered intravenously once daily during a period of at least 10 consecutive days.

The deproteinised calf blood preparation may be administered on consecutive days e.g. if the patient suffering from diabetes mellitus is hospitalised. Alternatively, the deproteinised calf blood preparation is administered in a nonconsecutive manner, e.g. during weekdays only. If so, it is preferable that the treatment schedule continues until the patient has received in total 20 doses. However, embodiments wherein the patient has received in total 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 doses or even more by parenteral administration are within the scope of the present invention. The parenteral administration may be followed by a period of enteral administration as outlined in the following.

The present invention provides a deproteinised calf blood preparation for use in prevention or treatment of diabetic peripheral polyneuropathy (DPN), wherein the deproteinised calf blood preparation is administered intravenously daily during a period of at least 10 consecutive days, 11-15 days, 16-20 days, 21-25 days, 26-30 days, 31-35 days or 36-38 days, such as 10 days, 14 days, 20 days, 25 days, 30 days, 36 days, 37 days or 38 days, followed by a period of enteral, preferably oral, administration. In preferred embodiments, the patient receives 11-15, 16-20, 21-25, 26-30, 36, 37 or 38 doses by enteral administration.

One embodiment of the invention provides a deproteinised calf blood preparation for use in prevention or treatment of diabetic peripheral polyneuropathy (DPN), wherein the deproteinised calf blood preparation is administered intravenously daily during a period of at least 10 consecutive days such as 14 days, 20 days, or 30 days. Most preferably, the deprotenised calf blood preparation is administered intravenously daily for a period of 20 days. In another preferred embodiment, 20 doses of deproteinised calf blood preparation is administered intravenously. It may be administered once, twice or three times daily or as a slow continuous infusion.

Generally, a dose of about 100 ml, 200 ml, 250 ml, 300 ml, 400 ml or 500 ml of the deproteinised calf blood preparation is administered. In a preferred embodiment, about 250 ml of deprotenised calf blood preparation having a content of active substances of about 4 mg/ml or about 8 mg/ml is administered intravenously once daily, i.e. doses of about 1000 mg or 2000 mg.

In some embodiments, this period of intravenous administration daily is followed by a period of oral administration in order to maintain and further improve the results obtained. The daily administration may comprise about 200 mg, 400 mg or 600 mg of the deproteinised calf blood preparation which may be administered once, twice or three times daily. In one embodiment, about 400 mg of the deproteinised calf blood preparation is administered orally three times daily giving a total of 1200 mg. In a preferred embodiment, about 600 mg of the deproteinised calf blood preparation is administered orally three times daily giving a total of 1800 mg daily.

The deprotenised calf blood preparation may be administered orally for a period of 10-20 days, 21-30 days, 31-40 days, 41-50 days, 51-60 days, 61-70 days, 71-80 days, 81-90 days, 91-100 days, 101-110 days, 111-120 days, 121-130 days, 131-140 days, 141-150 days, 151-160 days or 161-170 days such as 10 days, 14 days, 17 days, 20 days, 30 days, 45 days, 60 days, 90 days, 120 days, 125 days, 130 days, 135 days, 140 days, 144 days, 145 days, 146 days, 150 days, 155 days, 160 days, 169 days, 170 days, 180 days or even longer as a substantially continuous treatment.

In a presently preferred embodiment the patient receives orally in total 10-20 doses, 21-30 doses, 31-40 doses, 41-50 doses, 51-60 doses, 61-70 doses, 71-80 doses, 81-90 doses, 91-100 doses, 101-110 doses, 111-120 doses, 121-130 doses, 131-140 doses, 141-150 doses, 151-160 doses or 161-170 doses, such as 10 doses, 14 doses, 17 doses, 20 doses, 30 doses, 45 doses, 60 doses, 90 doses, 120 doses, 125 doses, 130 doses, 135 doses, 140 doses, 144 doses, 145 doses, 146 doses, 150 doses, 155 doses, 160 doses, 169 doses, 170 doses, 180 doses or even more as a substantially continuous treatment.

As evidenced in the following, it has surprisingly been found that a preparation of the invention is effective in reducing Total Symptoms Score in diabetic peripheral polyneuropathy (DPN). Thus, a disclosure of the invention relates to a deproteinised calf blood preparation for use in reducing Total Symptoms Score in diabetic peripheral polyneuropathy (DPN), wherein the deprotenised calf blood preparation is administered according to the invention.

A further disclosure relates to a deproteinised calf blood preparation for use in lowering the average of the Total Symptoms Score in diabetic peripheral polyneuropathy (DPN) in a group of patients suffering from diabetes mellitus, wherein the deproteinised calf blood preparation is administered according to the invention. The deproteinised calf blood preparation for use according to the invention may be particularly useful when the patient is a non-drinker, non-smoker or both.

An important disclosure of the present invention relates to a deproteinised calf blood preparation for use in reducing the intensity or frequency of one or more of the individual symptoms of lancinating pain, burning pain, prickling (paresthesia) or asleep numbness in a patient suffering from diabetes mellitus, wherein the deproteinised calf blood preparation is administered according to the invention.

Another important disclosure of the present invention relates to a deproteinised calf blood preparation for use in reducing the Vibration Perception Threshold in a patient suffering from diabetes mellitus, wherein the deproteinised calf blood preparation is administered according to the invention.

A further important disclosure of the present invention relates to a deproteinised calf blood preparation for use in improving mental health as assessed by SF36 in a patient suffering from diabetes mellitus, wherein the deproteinised calf blood preparation is administered according to the invention.

Other disclosures of the invention relate to a deproteinised calf blood preparation for use in improving the physical health e.g. by reducing various symptoms as described in detail elsewhere. In a disclosure the invention relates to a deproteinised calf blood preparation for use in improving the mental and/or physical health, i.e. the Quality of Life (QoL), for example as evaluated by the SF 36 Health Survey questionnaire.

Yet another aspect of the present invention is to provide use of a deproteinised calf blood preparation for the preparation of a medicament for the prevention or treatment of diabetic peripheral polyneuropathy (DPN), wherein the deproteinised calf blood preparation is to be administered according to the invention.

Still another aspect of the present invention relates to the use of a deproteinised calf blood preparation for use in a method for prevention or treatment of diabetic peripheral polyneuropathy (DPN) in a patient suffering from diabetes mellitus, said method comprising administering the deproteinised calf blood preparation according to the invention.

An embodiment of the invention relates to a deproteinised calf blood preparation for use in prevention or treatment of diabetic peripheral polyneuropathy, wherein the deproteinised calf blood preparation is administered parenterally at least once and wherein the parenteral administration is followed by a period of enteral administration.

Another embodiment of the invention relates to a deproteinised calf blood preparation according to the invention wherein the deproteinised calf blood preparation is administered intravenously once daily during a period of at least 10 consecutive days.

Yet another embodiment of the invention relates to a deproteinised calf blood preparation according to the invention, wherein the deproteinised calf blood preparation is administered intravenously once daily for a period of 20 days.

Still another embodiment of the invention relates to a deproteinised calf blood preparation according to the invention, wherein 20 doses of deproteinised calf blood preparation is administered intravenously.

The invention includes embodiments wherein the dose of the deproteinised calf blood preparation is about 1000 mg or about 2000 mg. These doses are preferably administered parenterally, such as intravenously.

When administered orally, a preferred embodiment of the invention relates to doses of about 600 mg of the deproteinised calf blood preparation which is to be administered orally three times daily.

Preferably, the deproteinised calf blood preparation is administered orally three times daily for 140 days.

Another disclosure of the invention relates to a deproteinised calf blood preparation for use in prevention or treatment of diabetic peripheral polyneuropathy by lowering the average of the Total Symptoms Score in diabetic peripheral polyneuropathy in a group of patients suffering from diabetes mellitus, wherein the deproteinised calf blood preparation is administered according to the invention.

Yet another disclosure of the invention relates to a deproteinised calf blood preparation for use in prevention or treatment of diabetic peripheral polyneuropathy by reducing the intensity or frequency of one or more of the individual symptoms of lancinating pain, burning pain, prickling or asleep numbness in a patient suffering from diabetes mellitus, wherein the deproteinised calf blood preparation is administered according to the invention.

Other disclosures of the invention relate to a deproteinised calf blood preparation for use in prevention or treatment of diabetic peripheral polyneuropathy by reducing the Vibration Perception Threshold in a patient suffering from diabetes mellitus, wherein the deproteinised calf blood preparation is administered according to the invention.

A further other disclosure of the invention relates to a deproteinised calf blood preparation for use in prevention or treatment of diabetic peripheral polyneuropathy by lowering the average of the Vibration Perception Threshold in diabetic peripheral polyneuropathy in a group of patients suffering from diabetes mellitus, wherein the deproteinised calf blood preparation is administered according to the invention.

One embodiment of the invention relates to a deproteinised calf blood preparation for use in prevention or treatment of diabetic peripheral polyneuropathy, wherein the deproteinised calf blood preparation is administered intravenously daily during a period of at least 10 consecutive days.

Another embodiment of the invention relates to the use of a deproteinised calf blood preparation for the preparation of a medicament for the prevention or treatment of diabetic peripheral polyneuropathy, wherein the deproteinised calf blood preparation is to be administered intravenously daily during a period of at least 10 consecutive days.

In a preferred embodiment of the invention a deproteinised calf blood preparation is used in prevention or treatment of diabetic peripheral polyneuropathy, wherein the period of intravenous administration is followed by a period of oral administration.

A further embodiment of the invention relates to the use of a deproteinised calf blood preparation for the preparation of a medicament for the prevention or treatment of diabetic peripheral polyneuropathy, wherein the period of intravenous administration is to be followed by a period of oral administration.

Another embodiment of the invention relates to a deprotenised calf blood preparation according to the invention, wherein about 250 ml of the preparation is administered intravenously once daily.

A further embodiment of the invention relates to the use of a deprotenised calf blood preparation for the preparation of a medicament for the prevention or treatment of diabetic peripheral polyneuropathy, wherein about 250 ml of the preparation is to be administered intravenously once daily.

Another embodiment of the invention relates to a deprotenised calf blood preparation according to the invention, wherein the preparation is administered intravenously once daily for a period of 20 days.

A further embodiment of the invention relates to the use of a deprotenised calf blood preparation according to the invention, for the preparation of a medicament for the prevention or treatment of diabetic peripheral polyneuropathy, wherein the preparation is to be administered intravenously once daily for a period of 20 days.

Another embodiment of the invention relates to a deprotenised calf blood preparation according to the invention, wherein about 600 mg of the deproteinised calf blood preparation is administered orally three times daily.

A further embodiment of the invention relates to the use of a deprotenised calf blood preparation according to the invention, for the preparation of a medicament for the prevention or treatment of diabetic peripheral polyneuropathy, wherein about 600 mg of the deproteinised calf blood preparation is to be administered orally three times daily.

Another embodiment of the invention relates to a deprotenised calf blood preparation according to the invention, wherein the deproteinised calf blood preparation is administered orally three times daily for 140 days.

A further embodiment of the invention relates to the use of a deprotenised calf blood preparation according to the invention, for the preparation of a medicament for the prevention or treatment of diabetic peripheral polyneuropathy wherein the deproteinised calf blood preparation is to be administered orally three times daily for 140 days.

Yet a further disclosure of the invention relates to the use of a deproteinised calf blood preparation for the preparation of a medicament for the prevention or treatment of diabetic peripheral polyneuropathy by reducing Total Symptoms Score in diabetic peripheral polyneuropathy, wherein the deproteinised calf blood preparation is to be administered according to the invention.

Another disclosure of the invention relates to the use of a deproteinised calf blood preparation for the preparation of a medicament for the prevention or treatment of diabetic peripheral polyneuropathy by lowering the average of the Total Symptoms Score in diabetic peripheral polyneuropathy in a group of patients suffering from diabetes mellitus, wherein the deproteinised calf blood preparation is to be administered according to the invention.

In some disclosures of the invention the patient is a non-drinker.
In disclosures of the invention the patient is a non-smoker. Evidently, the patient may also be both a non-drinker and a non-smoker.

One disclosure of the invention relates to the use of a deproteinised calf blood preparation for the preparation of a medicament for the prevention or treatment of diabetic peripheral polyneuropathy by reducing the intensity or frequency of one or more of the individual symptoms of lancinating pain, burning pain, prickling or asleep numbness in a patient suffering from diabetes mellitus, wherein the deproteinised calf blood preparation is to be administered according to the invention.

Another disclosure of the invention relates to a deproteinised calf blood preparation for use in reducing the Vibration Perception Threshold in a patient suffering from diabetes mellitus, wherein the deproteinised calf blood preparation is administered according to the invention.

A further disclosure of the invention relates to the use of a deproteinised calf blood preparation for the preparation of a medicament for the prevention or treatment of diabetic peripheral polyneuropathy by reducing the Vibration Perception Threshold in a patient suffering from diabetes mellitus, wherein the deproteinised calf blood preparation is to be administered according to the invention.

Another important disclosure of the invention relates to a deproteinised calf blood preparation for use in lowering the average of the Vibration Perception Threshold in diabetic peripheral polyneuropathy in a group of patients suffering from diabetes mellitus, wherein the deproteinised calf blood preparation is administered according to the invention.

A further disclosure of the invention relates to the use of a deproteinised calf blood preparation for the preparation of a medicament for the prevention or treatment of diabetic peripheral polyneuropathy by lowering the average of the Vibration Perception Threshold in diabetic peripheral polyneuropathy in a group of patients suffering from diabetes mellitus, wherein the deproteinised calf blood preparation is to be administered according to the invention.

Another disclosure of the invention relates to a deproteinised calf blood preparation for use in improving mental health as assessed by SF36 in a patient suffering from diabetes mellitus, wherein the deproteinised calf blood preparation is administered according to the invention.

### Brief description of the figures and legends:

Figure 1: Disposition of patients.

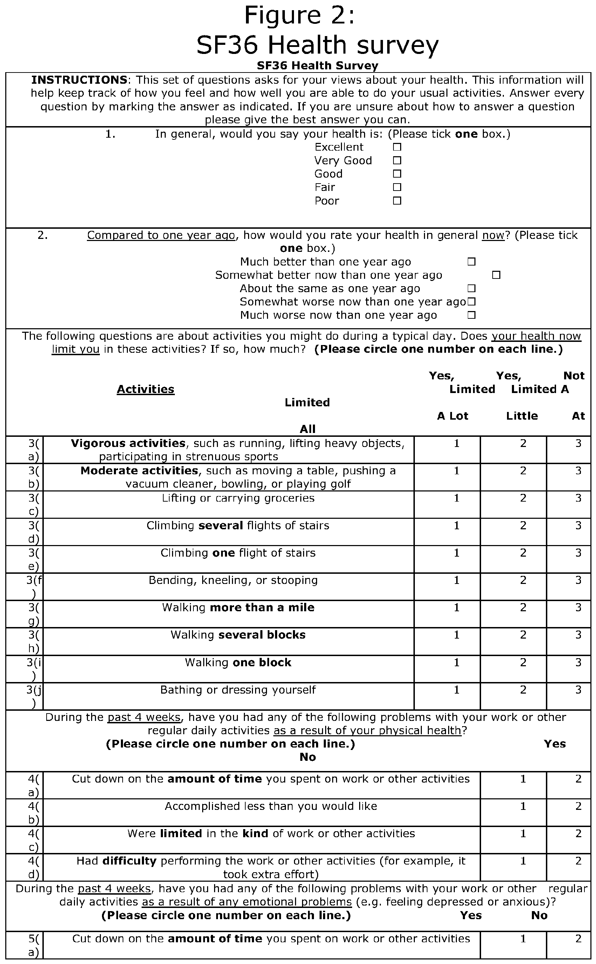

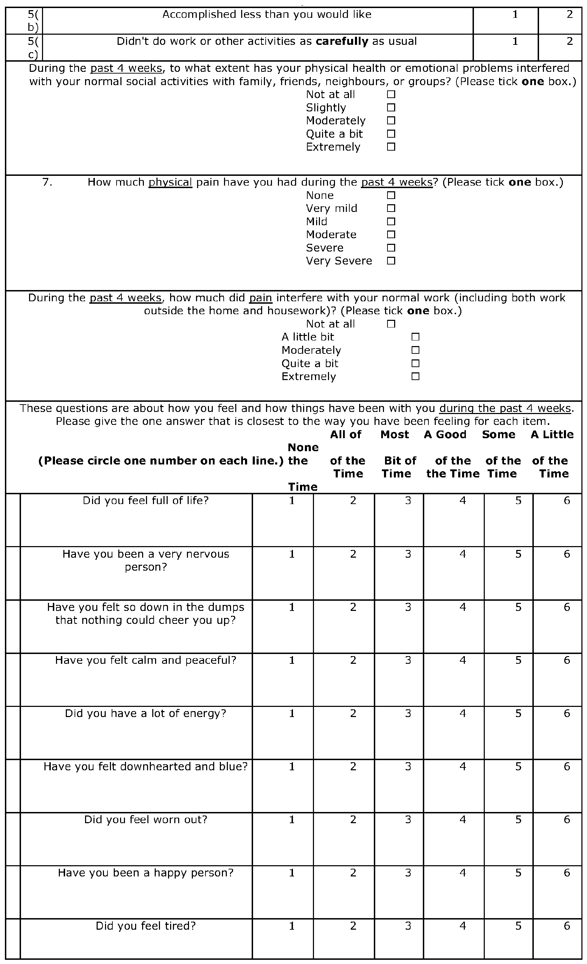

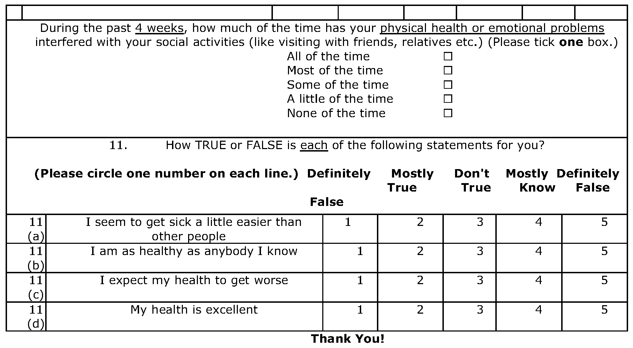

Figure 2: SF36 Health survey
Figure 3: TSS (feet).
Figure 4: Total symptom score (TSS) during treatment with Actovegin® (n=281) or placebo (n=286).
Figure 5: Proportion of patients achieving a TSS reduction of X % or more.
Figure 6: Proportion of patients achieving a TSS reduction of X units or more.
Figure 7: VPT evaluation scheme.
Figure 8: Vibration perception threshold (VPT) during treatment with Actovegin® (n=281) or placebo (n=286).
Figure 9: The proportion of patients achieving a VPT Reduction of X % or more.
Figure 10: The proportion of patients achieving a VPT reduction of X units or more.
Figure 11: The NIS - LL evaluation scheme
Figure 12: The mean NIS-LL profiles by treatment, ITT
Figure 13: The proportion of patients achieving a NIS-LL Reduction of X % or more.
Figure 14: The proportion of patients achieving a NIS-LL Reduction of X units or more.

The present invention will now be described in more detail in the following.

### Detailed description of the invention

Prior to discussing the present invention in further details, the following terms will first be described and defined as appropriate:
Parenteral administration: The term parenteral administration includes any relevant parenteral administration form such as intravenous, intramuscular, or subcutaneous administration.

Enteral administration: The term enteral administration includes any relevant enteral administration form such as peroral administration in solid or liquid form or administration through the anal canal e.g. by means of a suppository.

Deproteinised calf blood preparations are well known to the person of skill in the art. Examples of how to prepare such preparations are outlined in the examples. A preferred calf blood preparation is commercially available as the medicament Actovegin®. In the present application, Actovegin® is used as a synonym for deproteinised calf blood preparation.
The term "diabetic peripheral polyneuropathy" herein also referred to as DPN is also well known to the person of skill in the art and further described in the section Background of the invention and the references cited therein. This complication may affect both type 1 and type 2 diabetic patients and thus, the present invention is relevant for both patient groups. However, the present invention is considered particularly relevant for patients suffering from type 2 diabetes mellitus.

By the term "prevention or treatment of diabetic peripheral polyneuropathy (DPN)" is in the present context meant that one or more of the symptoms of diabetic peripheral polyneuropathy (DPN), such as the symptoms outlined in the Total Symptom Score (TSS) or the Vibration Perception Threshold (VPT) are reduced by the treatment when compared to placebo treatment in a statistically significant manner. These symptoms are generally recognised within the art (Ziegler D et al, Diabetes Care, vol. 22 (8), p 1296-1301 and Abbott CA et al. Diabetes Care 1998, vol. 21 (7), 1071-1075) but also described in detail below.

### Total Symptom Score (TSS)

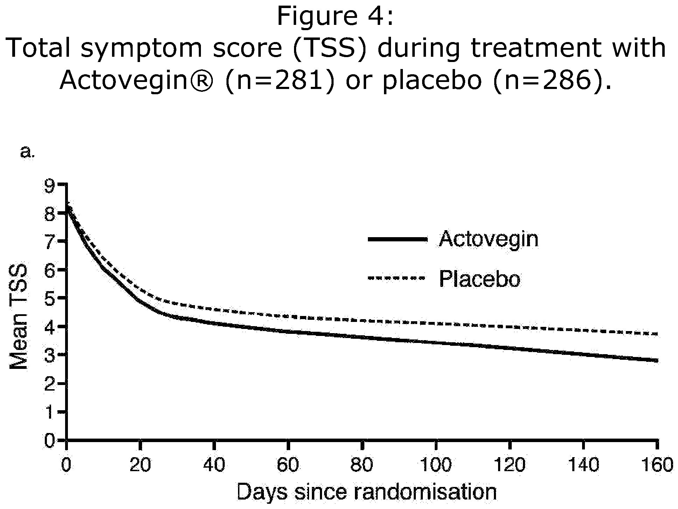

Total Symptom Score (TSS) comprises 4 individual symptoms: lancinating pain, burning pain, prickling (parestesia) and asleep numbness. Neuropathic symptoms in the foot (lancinating pain, burning pain, prickling (paresthesia) or asleep numbness) are scored ranging from 0 (0 symptoms) to a maximum of 14.64 (all symptoms are severe and [almost] continuously present). Thus, TSS is a bidimensional summation of the severity and frequency of the four main positive neuropathic sensory symptoms (Ziegler D et al., Diabetologia 38: 1425-1433, 1995). A scoring scheme wherein it is indicated whether the patient over the past 24 hours have experienced symptoms is outlined in the examples. As evident from the scheme (figure 4), the intensity is scored as mild (1), moderate (2) or severe (3) summing up to a maximum of 12. To this score is added the frequency of the individual symptoms as occasionally (0), often (0.33) or continuously (0.66) summing up to a maximum of 2.64. Thus, the maximum score is 14.64.

### Vibration Perception Treshold (VPT)

A primary endpoint was Vibration Perception Threshold (VPT). Vibration Perception Threshold (VPT) is a predictor for future risk of foot ulceration. Vibration Perception Treshold (VPT) is assessed at five different locations of both feet (med. malleol (the medial malleolus), med.cap.1. met (medical head of the 1^{st} metatarsal bone), hallux pulpa (pulp of the great toe), lat.cap.5.met. (lateral head of the 5^{th} metatarsal bone), and tub.oss.5 met. (tuberosity of the 5^{th} metatarsal bone) in duplicate using a neurothesiometer also referred to as a biothesiometer (Biomedical Instrument Company, Newbury, Ohio, USA). The scores of the five measurements were averaged for each foot. The two scores were treated as repeat measurements in the statistical model.

Other measurements have been assessed in the clinical trial such as the following:
Neuropathy Impairment Score of the lower limbs (NIS-LL)
   The Neuropathy Impairment Score of Lower Limbs (NIS-LL) is used to evaluate the effect of the deproteinised calf blood preparation on the neuropathic deficit (impairment). A standard group of muscles (hip, knee, ankle, toe) is evaluated for weakness and reflex activity (quadriceps, ankle) and sensory activity such as touch-pressure, pinprick, vibration and joint position are graded as described by Vera Bril, European Neurology, 1999, 41 (suppl. 1) 8-13.

### Quality of Life (QoL)

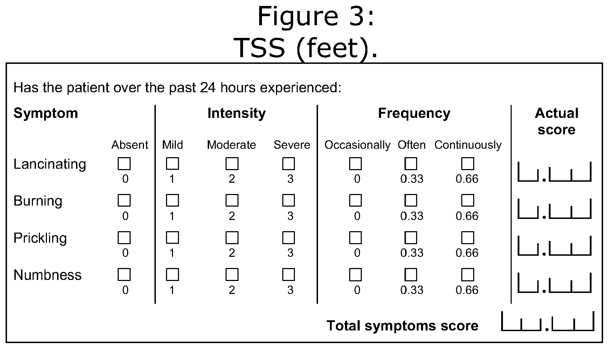

Quality of Life (QoL) is measured as outlined in the SF36 Health survey (fig. 3). SF-36 is defined as short form questionnaires used in type 2 diabetic populations (Luscombe F.A. Value in Health, vol. 3, suppl. 1, 2000, p. 15-28).

By the abbreviation "ITT" is meant "intention to treat" and is used about the population of patients who were exposed to the study medication (ITT population).

By the abbreviation "PP" is meant "per protocol". Thus, e.g. "PP analysis set" means all patients which have completed the treatment period according to the clinical trial protocol.

The present invention is based upon the results of a multi-center, double-blind, placebo-controlled, randomised, parallel group clinical trial to evaluate efficacy and safety of a deproteinised calf blood preparation, Actovegin®, in diabetic type 2 patients with symptomatic diabetic peripheral polyneuropathy (DPN). In total 26 centres in Russia, Ukraine, Kazakhstan have been involved and the total number of randomised patients is 569.

Treatment with a deproteinised calf blood preparation by administration of 250 ml of the deprotenised calf blood preparation intravenously once daily for 20 days followed by oral treatment of 600 mg of the deprotenised calf blood preparation three times daily for 140 days resulted in improved neuropathic symptoms and signs in diabetic patients with peripheral polyneuropathy (DPN). Further, the Quality of Life (Mental Health) showed significant improvement in the treatment group compared to the placebo group. The safety profile was the same in the treatment group and the placebo group.

By the abbreviation "v" is meant "visit at clinical trial center e.g. "v21" means visit at the clinical trial center after the patient had received 21 dosages.

More specifically, the results of the clinical trial showed that Total Symptom Score (TSS) decreased from 8.3 at screening to 4.4 at end of infusion (v21) and to 2.8 at end of trial (v26) in the treatment group, whereas in the placebo group the TSS decreased from 8.4 at screening to 4.9 at v21 and to 3.7 at v26. Thus, the TSS was reduced from 8,3 to 2,8 in the treated group equalling a reduction of 5,5 points or 66%. The mean changes from screening to v21 and v26 were -3.8 and - 5.5 for the treatment group and -3.6 and -4.7 for the placebo group.

This improvement in Total Symptom Score (TSS) was shown to be significantly larger in the treatment group (p<0.0001) compared to the placebo group. TSS_{average} (herein also referred to as TSS_{AUC}) was lower for the treatment group (3.9) than for the placebo group (4.6); again, the difference was significant (p=0.0005). TSS_{average} is defined as the average of TSS over the time course of the trial. It is calculated as the area under the curve (AUC) divided by the number of days of exposure. Thus, it can be called TSS_{AUC} and can be used interchangeably with TSS_{average}.

VTP_{average} is defined the same way as the TSS_{average} but was log transformed. NIS-LL_{average} is defined the same way as the TSS_{average} and was analysed in the same way as the primary endpoint TSS_{average}.

By the mathematical abbreviation "mean" is meant the measure of the middle value of a data set as commonly known by a person skilled in the art. It is calculated as the sum of all individual values divided by the total number (n) of all those individual values.

By the mathematical abbreviation "SD" is meant the standard deviation. Standard deviation is a measure of the variability or dispersion of a data set, or a probability distribution. A low standard deviation indicates that the data points tend to be very close to the same value (the mean), while high standard deviation indicates that the data are "spread out" over a large range of values.

Smoking and alcohol use are considered to influence the severity of DPN. Smoking and drinking habits of the study population were as follows: 13% of the patients were smokers with a weekly mean use of 73 units. Non-smokers never smoked. 44% of the patients never drank alcohol, whereas 38% took alcohol monthly at the most and 15% had alcohol 2 to 4 times a month.
From the results it appears that the deproteinised calf blood preparation is moderately more effective than placebo in drinkers and much more effective than placebo in non-drinkers. Also, it appears that the deproteinised calf blood preparation is moderately more effective than placebo in smokers and much more effective than placebo in non-smokers. Thus, the deproteinised calf blood preparation appears to be particularly useful in treatment of non-drinkers and non-smokers but it is also of value in patients who drink or smoke or both.

For all of the individual symptoms of TSS there was a significant improvement in the treatment group over the placebo group, i.e. reduction in the intensity or frequency of one or more of the individual symptoms of lancinating pain, burning pain, prickling or asleep numbness.

The results with regard to Vibration Perception Threshold (VPT) showed that VPT decreased from 19.7 at screening to 17.4 at v21 (end of infusion) and to 16.2 at v26 (end of trial) in the treatment group whereas in the placebo group VPT decreased from 20.0 at screening to 17.9 at v21 and to 17.2 at v26. The mean changes from screening to v21 and v26 were -2.4 and -3.6 for the group treated with the deproteinised calf blood preparation and -3.2 (-2.2 upon later calculations) and -2.9 for the placebo group. This improvement in VPT was significantly larger in group treated with the deproteinised calf blood preparation (p=0.01, upon later calculations p=0.017)).

VPTaverage was lower for the treatment group (2.78) than for the placebo group (2.81), and the difference reached borderline significance (p=0.07).

Quality of Life improved in the group treated with the deproteinised calf blood preparation. Physical health increased from 39.8 at screening to 42.9 at v21 and to 44.4 at v26 and mental health increased from 39.8 at screening to 44.5 at v21 and to 45.3 at v26.

In the placebo group physical health increased from 39.9 at screening to 42.1 at v21 and to 43.1 at v26 and mental health increased from 39.9 at screening to 43.3 at v21 and to 43.6 at v26.

The effect of treatment with the deproteinised calf blood preparation on Mental Health was significant (p=0.028).

The results obtained are surprising in view of the results reported in the prior art.

An adverse event (AE) is defined as any untoward medical occurrence in a patient or clinical trial subject administered a medicinal product and which does not necessarily have a causal relationship with this treatment.

The following should not be recorded as an AE, if recorded at screening:
- A pre-planned procedure, unless the condition for which the procedure was planned has worsened since screening
- A pre-existing condition found as a result of screening procedures. Complications to pre-planned procedures should be reported as AEs.

A serious adverse event (SAE) is defined as any untoward medical occurrence that at any dose
- results in death
- is life-threatening. Life-threatening in the definition of an SAE refers to an event in which the subject was at risk of death at the time of the event. It does not refer to an event which hypothetically might have caused death if it was more severe
- requires hospitalisation or prolongation of existing hospitalisation
- results in persistent or significant disability/incapacity
- is a congenital anomaly/birth defect
- is a medical important AE that is not immediately life-threatening or does not result in death or require hospitalisation but may jeopardise the subject or may require intervention to prevent one of the other outcomes listed in the definition above.

A non-serious AE is defined as any AE that does not meet the criteria of an SAE.

Safety analysis did not reveal any relevant differences in treatment-emergent AEs (TEAEs) or serious AEs (SAEs) between groups.

The invention will now be described in further details in the following non-limiting examples.

### Examples

The examples that follow are divided into examples relating to the preparation of different deproteinised calf blood preparations: 1) preparation of solution for intravenous administration, 2) granules, 3) use of granules for manufacture of solid dosage forms, and 4) results obtained from a clinical trial using deproteinised calf blood preparation Actovegin®.

### Example 1

### 1) Preparation of solution for intravenous administration

Pharmaceutical preparations which contain extract substances from deproteinised calf blood are commercially available under the name "Actovegin®" from Hormonchemie, Munich, or under the label "Actihaemyl" from Solko, Basel.

Various methods for making deproteinised calf blood preparations are described in the art. German Pat. No. 1,076,888 describes the production of injectable preparations of a concentration of 30 to 60 mg of dry matter/ml of solution by concentrating solutions of the extract substances. Gentle drying methods are to be considered for obtaining the dry matter, even for the production of solid medicament forms, because of the high thermal instability of the extract substances. Austrian Pat. No. 330,953 has disclosed a method for the production of dry preparations from calves blood extracts, wherein the extract substances are mixed with an adsorbent, for example highly dispersed silica, whereupon the resulting thixotropic gel is dried in vacuo.

Generally, deproteinised calf blood preparations like Actovegin® are obtained by subjecting calf blood to standard methods known to the skilled person. A not-exhaustive list of suitable methods includes dilution, filtration such as ultrafiltration, precipitation, pH adjustment, purification steps such as adsorption on active carbon and/or heat treatment. This raw deproteinised calf blood preparation (bulk solution) made by such methods may then be used as a basis for further formulation such as liquid formulations suitable for intraveneous administration and formulations useful for peroral administration.

For preparation of a formulation suitable for intravenous administration the bulk solution may further be subjected to dilution, adjustment of pH and isotonicity (NaCl), sterile filtration and aseptic filling on bottles, and heat treatment, if desired.

In the following the preparation of two solutions for intravenous administration is disclosed.
A) For the preparation of a 10% NaCl infusion product, (4 mg/ml), 250 ml bottles suitable for intravenous administration, an appropriate amount of the bulk solution is mixed with water for injection and thereafter pH and isotonically adjusted by using NaCl, hydrochloric acid, phosphate buffer and salts.
   The resulting solution is filtrated using a pore size of 0,45 µM, followed by a sterile filtration step using a pore size of 0,2 µM. Then the solution is aseptically filled onto 250 ml injection bottles, closed with sterilized rubber stoppers and heat treated at 112° C for 30 minutes. The bottles are visually inspected for impurities, labelled and packed.
B) For the preparation of a 20% NaCl infusion product (8 mg/ml), 250 ml bottles suitable for intravenous administration, an appropriate amount of the bulk solution is mixed with water for injection and thereafter pH and isotonically adjusted by using NaCl, hydrochloric acid, phosphate buffer and salts.
   The resulting solution is filtrated using a pore size of 0,45 µM, followed by a sterile filtration step using a pore size of 0,2 µM. Then the solution is aseptically filled onto 250 ml injection bottles, closed with sterilized rubber stoppers and heat treated at 112° C for 30 minutes. The bottles are visually inspected for impurities, labelled and packed.

### Example 2:

### 2) Granules

For preparation of solid formulation, fluidized-bed methods could be employed. A preferred method for simultaneously obtaining the dry matter from aqueous solutions of extract substances from deproteinized calves blood and producing granules which can be compressed to give solid medicament forms and contain at least 50% by weight of extract substances, relative to the total dry weight of the granules, and to the use of granules, obtained by this method, for producing pharmaceutical preparations which contain, as the active substance, the extract substances from deproteinized calves blood.

An improved alternative for adsorbing the active substances for blood extracts on solid materials is, according to Austrian Pat. No. 330,953, to obtain the dry matter by freeze-drying (lyophilization) of approximately 5 to 10% aqueous solutions of the extract substances. Apart from the fact that freeze-drying of relatively large volumes of the aqueous solutions consumes a lot of time and energy, freeze-drying has the disadvantage that the lyophilizates obtained represent a very fine and highly hygroscopic powder which is unsuitable for the production of solid medicament forms by direct tabletting or by dry granulation and subsequent compression.

Granules suitable for tabletting have hitherto been produced from the lyophilizate by subsequent moist granulation with polyvinylpyrrolidone as a binder and carboxymethylstarch as a filler, but water cannot be used as the granulating fluid and, instead, organic solvents, for example isopropanol, must be used. Because of their toxicity, however, organic solvents are only used reluctantly for the production of medicament forms which can be administered orally. Moreover, the hygroscopic lyophilizate absorbs moisture from the surrounding air during the granulation process, so that it is necessary either to operate with air-conditioning at a relatively low atmospheric humidity or to subject the finished granules to a drying process at about 40° C for several hours, the extract substances being exposed to a further temperature stress which adversely affects the quality and leads to a discoloration of the finished preparations on storage.

In order to improve both the method for obtaining the dry matter from the aqueous solutions arising in the isolation of the extract substances and the production of granules which can be compressed to give solid medicament forms, while avoiding the abovementioned disadvantages, a uniform improved product quality being an essential requirement in addition to economic advantages. In particular, the dry matter should be obtained under mild conditions during the entire production process and granules ready for compression should be produced with a high weight fraction of extract substances, these granules being processable to give solid medicament forms of high stability, which do not show discolouration even on prolonged storage at ambient temperatures and have more constant quality parameters.

This object is achieved by a method for simultaneously obtaining the dry matter from aqueous solutions of extract substances from deproteinized calves blood and producing granules which can be compressed to give solid medicament forms and contain at least 50% by weight of extract substances, relative to the total dry weight of the granules, a binder and pharmaceutically acceptable fillers and carriers, in a continuous process comprising the steps of:
(a) placing a given quantity of the fillers and carriers into a fluidized-bed granulator and establishing a uniform fluidized bed by introducing a flow of fluidizing gas,
(b) spraying a volume, containing the desired quantity of extract substances, of a concentrated aqueous solution of the extract substances, which additionally contains 0.2 to 2 parts by weight of the binder in the dissolved state per 10 parts by weight of extract substances, on to the fillers and carriers in the fluidized bed, the temperature of the fluidizing gas flowing in and the spraying rate being adjusted, during the spraying-in of the total solution volume or at least during the major part thereof, in such a way that, at a fluidized-bed temperature of 25° C to 30° C and at a water content of 15 to 20% by weight in the fluidized bed, the quantity of water sprayed in and that being evaporated approximately correspond to each other, no shapes are formed and the fluidized-bed material remains pulverulent, and
(c) agglomerating and granulating the mixed material thus obtained by increasing the water content of the fluidized bed to 35 to 45% by weight, while the solution of the extract substances is still being sprayed in or after completion thereof, until the desired granule size has been reached, and briefly drying the resulting granules at a fluidized-bed temperature of 30° C to at most 45° C by increasing the temperature of the fluidizing gas flowing in.

The advantages of this method are, on the onehand, an improvement in the product quality of the granules obtained which, after discharge from the fluidized-bed granulator, can be compressed either directly or after the addition of further pharmaceutically acceptable auxiliaries to give readily storable solid medicament forms of high stability, perfect constant weight and excellent galenic properties. On the other hand, the method makes it possible both to obtain the dry matter under mild conditions from large volumes of aqueous solutions of extract substances from deproteinized calves blood and to produce granules ready for compression in a single continuous process in a substantially simpler and more economical manner than has hitherto been the case with the known method.

Commercially available fluidized-bed granulators are suitable for carrying out this method. Such fluidized-bed granulators consist essentially of a material container, into which the fluidizing gas flows from the side or from below, both the flow rate and the temperature of the fluidizing gas flowing in being controllable. These devices also have one or more sprayheads in the form of single-component or two-component nozzles, through which pure liquids and solutions can be sprayed into the material container at an adjustable spraying rate by means of a pump and which are arranged such that the atomized liquid or solution flows directly into the fluidized bed. A mechanical agitating or vibrating device on the container floor or a shaking device are intended to provide an additional mixing effect if required.

For carrying out the method in practice, a given quantity of the fillers and carriers, which depends on the desired weight proportion of these additives in the finished granules and is in every case less than 50% by weight, preferably 25 to 45% by weight, relative to the total dry weight of the finished granules, is placed into the material container and a uniform fluidized bed is formed by introducing the flow of fluidizing gas. Galenically conventional fillers and carriers of small particle size, which at the same time have a large surface area and are insoluble or only sparingly soluble in water, are above all suitable for producing the granules. As examples of suitable fillers and carriers having the properties described above, microcrystalline cellulose (commercially available, for example, under the name Avicel PH 101), cross-linked sodium carboxymethylcellulose (commercially available, for example, under the name Ac-Di-Sol), cross-linked polyvinylpyrrolidone (commercially available, for example, under the name Polyplasdone) or mixtures of these substances may be mentioned, and the mixing ratios can be selected as desired within a wide range and, here again, mixtures of equal parts by weight of these substances are preferred. Microcrystalline cellulose or cross-linked sodium carboxymethylcellulose have proven to be very particularly suitable for this purpose.

As the fluidizing gas, dry air can be used in a very simple manner without any disadvantage regarding the quality and stability of the extract substances. However, other gases which are inert towards the extract substances such as, for example, nitrogen, are of course also suitable for forming the fluidized bed. The total throughput of fluidizing gas per hour depends on the size of the fluidized-bed granulator and amounts to, for example, 500-3000 m³/h for a nominal volume of the material container of 60 liters. As soon as a uniform fluidized bed has formed due to the flow pressure, spraying of the aqueous solution of the extract substances can be started. For this purpose, concentrated aqueous solutions are advantageously used which contain up to 50% by weight, preferably 15 to 25% by weight, of extract substances and the volumes of which are calculated such that the total quantity of the extract substances sprayed on is at least 50% by weight, preferably 55-70% by weight, relative to the total dry weight of the finished granules.

These solutions additionally contain 0.2 to 2 parts by weight of a water-soluble binder, preferably 0.5 to 1 part by weight, per 10 parts by weight of extract substances. The weight proportion of the binder in the finished granules is then, for example 1 to 10% by weight, preferably 2 to 6% by weight, relative to the total dry weight of the granules.

Suitable binders for producing the granules are galenically conventional water-soluble binders, for example pregelatinized starch, water-soluble cellulose, such as methylcellulose, hydroxypropylcellulose, hydroxypropylmethylcellulose or other water-soluble high-molecular compounds such as polyvinyl alcohol, polyvinylpyrrolidone and the like. Polymers of 1-vinyl-2-pyrrolidone, which are commercially available, for example, under the name Kollidon K 25 or Kollidon 90, are used with particular preference.

The temperature of the fluidizing gas flowing in during the spraying process and the spraying rate, at which the aqueous solution of the extract substances is sprayed together with the binder on to the fluidized fillers and carriers, depends on the quantity of the auxiliaries originally introduced and on the nominal volume of the material container of the fluidized-bed granulator. In this phase of the method, the temperature and the spraying rate are matched such that, at a fluidized-bed temperature of 25° C to 30 °C, a steady water content of 15 to 20% by weight, relative to the total weight, is established in the fluidized-bed material and the quantity of water sprayed in and that being evaporated approximately correspond to each other.

For this purpose, fluidizing gas temperatures of about 30° C to 80° C are required, depending on the quantity of the fillers and carriers introduced and on the size of the material container.

Under the conditions indicated, shaping does not yet take place. The fillers and carriers remain capable of absorbing the extract substances which are advantageously not subjected to any significant temperature stress in the fluidized bed, while the major part of the water is removed, and a fine flowable mixed material is obtained which remains pulverulent in this first phase of the method and hardly tends to agglomerate.

The agglomeration of the pulverulent material for building up the granules to the desired size is obtained in the method by increasing the water content in the fluidized bed to 35 to 45% by weight, preferably to 38 to 40% by weight. For this purpose, the water content can be increased either during the spraying process itself, when the major part of the desired quantity of extract substances has already been sprayed on, or when spraying-on of the extract substances has been completed. In a particularly advantageous embodiment of the method, the major part of the solution, which contains the extract substances and the binder, for example 75 to 85% by volume, depending on the concentration of extract substances, of the total volume of the solution to be sprayed on, is sprayed on under the conditions which are indicated above and which do not lead to agglomeration of the mixed material, whereas, while the remaining part of the solution volume is sprayed on, the temperature of the fluidizing gas flowing in is lowered and/or the spraying rate is increased, so that a greater quantity of water is sprayed in than can be evaporated simultaneously and, consequently, the water content of the fluidized bed rises to the value required for agglomerating and granulating.

It is also possible, however, to spray on the total solution volume under conditions which do not lead to agglomeration, and then to increase the water content in the fluidized bed to the value required for granulation by spraying in pure water.

As soon as the water content in the fluidized bed exceeds a value of about 35% by weight, agglomeration starts and even, uniform granules are produced.

During the entire granulation step, a water content within the limits indicated above is maintained in the fluidized-bed material. Depending on the adjustment of the water content, granules which have a diameter in the range from 0.03 to 2 mm, preferably 0.05 to 1 mm, can be obtained reproducibly by this method.

When granules of the desired size have formed, the drying phase is started by raising the temperature of the fluidizing gas. The temperature of the fluidizing gas is here advantageously selected such that, for mild treatment of the extract substances, the granule temperature in the fluidized bed does not exceed 30° C to 32 °C. Drying is finished as soon as the granule temperature rises. To remove the residual water, the temperature of the granules can be allowed to rise for a short period, for example 5 to 15 minutes, to 40° C to 45° C, advantageously 40° C to 43 °C, without adversely affecting the stability of the extract substances and the quality of the granules. In this way, granules with a maximum water content of about 1.5 to 3% by weight, relative to the total weight, are obtained.

This method is efficient, simple and easily reproducible. The production of 58 kg of dry granules, ready for compression, from the aqueous concentrate of the extract substances from deproteinized calves blood takes, for example, only about 7 hours in continuous operation, whereas freeze-drying of the same quantity, carried out according to the state of the art, alone takes about 7 to 9 days.

The following example describes the manufacture of granules.
A) 3000 g of a mixture of equal parts by weight of Avicel PH 101 (microcrystalline cellulose) and Polyplasdone (cross-linked polyvinylpyrrolidone) are placed into the material container of a fluidized-bed granulator having a nominal volume of 7 liters, and a uniform fluidized bed is formed-by introducing a flow of dry air at a flow rate of 500 m³ per hour and a temperature of 80 °C
20 Liters of a 20% aqueous solution of the extract substances are mixed with 1 liter of 20% aqueous Kollidon solution, fed by means of a peristaltic pump to a two-component nozzle and sprayed into the fluidized bed. The spray rate is controlled such that a fluidized-bed temperature of 28° C to 30° C and a steady water content of the fluidized-bed material of about 15% by weight are established in the fluidized-bed granulator, the quantity of water sprayed in and that being evaporated being in balance and no agglomeration being observable.
The mean spray rate for maintaining these conditions is 70 g of solution per minute. After about 3/4 of the total solution volume have been sprayed in, the temperature of the inlet air is lowered to 30° C and the remainder of the solution volume is sprayed in at the same spray rate. The steady water content in the fluidized bed then rises and the agglomeration of the powder starts at a water content of 38 to 40% by weight. Granulation is continued at this water content until granules of a diameter from 0.06 mm to 0.8 mm have formed from the total powder quantity. After completion of spraying, the temperature of the inlet air is raised again to 60° C - 80° C and the drying phase is thus initiated. The temperature of the granules during drying is 30° C to 32° C Drying is complete when the temperature of the granules rises. To remove the residual water, the temperature of the granules is held for 15 minutes at 43° C This gives 7350 g of granules having the following properties:

| | |
|---|---|
| Content of extract substances | 544.2 mg/g of granules |
| Kollidon K 25 | 27.3 mg/g of granules |
| Avicel PH 101 | 204.0 mg/g of granules |
| Polyplasdone | 204.0 mg/g of granules |
| Residual water content | about 20 mg/g of granules, corresponding to about 2% |
| Diameter | from 0.08 to 0.8 mm |

B) Experiment, Example 1 A), is repeated, 3000 g of microcrystalline cellulose (Avicel I PH 101) being used as the filler or carrier and 1 liter of a 20% aqueous solution of Kollidon 90, 1-vinyl-2-pyrrolidone polymer, being added as the binder for the solution of the extract substances.
This gives granules having the following properties:

| | |
|---|---|
| Content of extract substances | 544.2 mg/g of granules |
| Kollidon 90 | 27.3 mg/g of granules |
| Avicel PH 101 | 408 mg/g of granules |
| Residual water content | about 20 mg/g of granules, corresponding to about 2% |
| Diameter | from 0.08 to 0.8 mm |

C) 15,000 g of Avicel PH 101 (microcrystalline cellulose) and 10,000 g AC-DI-Sol (cross-linked sodium carboxymethylcellulose) are placed into the material container of a fluidized-bed granulator having a nominal volume of 60 liters. A uniform fluidized bed is maintained by introducing air at a flow rate of 800 m³ /hour and at a temperature of 80° C 166.6 Liters of a 20% aqueous solution of the extract substances are mixed with a solution of 1660 g of Kollidon K 25 in 16.6 liters of water, fed in by means of a peristaltic pump (3 nozzles having a 1.2 mm nozzle orifice) and sprayed into the fluidized bed. At a spray rate of about 600 g of Actovegin®/Kollidon solution per minute, a steady water content of about 15% and a temperature of 30° C to 32° C are established in the fluidized bed.
After 140 liters have been sprayed in under these conditions, the inlet air temperature is lowered to room temperature, and the water content is thus increased and the agglomeration phase is initiated. After completion of spraying-in, the granules formed in the fluidized-bed granulator are dried at an inlet air temperature of 80 °C
The dried granules have the following properties:

| | |
|---|---|
| Bulk density | 0.53 g/cm³ |
| Tamped density | 0.63 g/cm³ |
| Flowability | 12.30 g/cm² second |
| Water content | 1.6% |
| Particle size | 60-500 µm |

D) 22,000 g Microcrystalline cellulose (Avicel PH 101) are placed into the material container of a fluidized-bed granulator having a nominal volume of 60 liters. A uniform fluidized bed is maintained by introducing air at a flow rate of 800 m³ /hour and at a temperature of 70° C 166.6 Liters of 20% aqueous solution of Actovegin® (extract substances from deproteinized calves blood) are mixed with a solution of 1660 g of Kollidon 90 (polymer of 1-vinyl-2pyrrolidone) in 16.6 liters of water, fed in by means of a peristaltic pump (3 nozzles having a 1.2 mm nozzle orifice) and sprayed into the fluidized bed. At an initial spray rate of about 600 g of Actovegin®/Kollidon 90 solution per minute, a steady water content of about 15% and a temperature of 30 °C-32° C are established in the fluidized bed.

After 2.5 hours at the same inlet air temperature, the spray rate is increased to 900 g/minute and the agglomeration phase is intensified. After the end of spraying-in, the granules formed are dried in the fluidized-bed granulator at an inlet air temperature of 70 °C.

The dried granules have the following properties:

| | |
|---|---|
| Bulk density | 0.53 g/cm³ |
| Tamped density | 0.63 g/cm³ |
| Flowability | 12.30 g/cm² second |
| Water content | 1.6% |
| Particle size | 60-500 µm |

### Example 3:

### 3) Use of granules for manufacture of solid dosage forms.

The granules produced have a high weight proportion of extract substances and, at uniform particle size, show excellent flow behavior, which allows them to be processed into solid medicament forms at perfect constant weight and high stability. The granules can be used for producing solid medicament forms which contain extract substances from deproteinized calves blood as the active substance. Preferably, compressed items, such as tablets, coated tablets, tablet cores or other compressed products of any desired shape and size, are produced. The granules can be compressed directly or after the admixture of further auxiliaries known in galenics, such as tablet binders, fillers, preservatives, tablet-disintegrating agents and the like. The nature and quantity of these auxiliaries depends on the desired mechanical strength and the dissolution rate of the compressed product.

Suitable examples of such auxiliaries for admixing before compression are stearates, such as magnesium stearate, calcium stearate and the like, or other lubricants conventionally used in tabletting, for example talc or glycerol esters of saturated natural fatty acids, such as are commercially available, for example, under the name Precirol, in a quantity of 1 to 10% by weight, relative to the total weight of the finished preparation.

The finished preparations contain, for example, 150 to 600 mg of extract substances per dose unit.

Solid medicament forms, produced with the use of the granules, do not show any discoloration even on prolonged storage at room temperature and have more constant quality parameters.

In the following use of the granules for manufacturing solid dosage forms is disclosed.

### A) Tablet cores.

7200 g of granules obtained according to Example 1, A) are mixed with 60 g of magnesium stearate and 40 g of talc and compressed in a rotary pelletting machine under a pressing power of 78.5-150 N/mm² to give tablet cores of 365 mg weight. This gives tablet cores having the following properties:

| Core properties: | | Composition: | |
|---|---|---|---|
| Diameter | 10.0 mm | Extract substances | 200 mg |
| Thickness | 5.5 mm | Polyplasdone | 75 mg |
| Breaking strength | 115 N | Avicel PH 101 | 75 mg |
| Attrition (4 min) | 20.1% | Kollidon K 25 | 10 mg |
| Disintegration | 8-10 min | Talc | 2 mg |
| | | Mg stearate | 3 mg |

These tablet cores have a constant content of extract substances and show no deterioration in quality or discoloration when stored for 24 months at room temperature.

### B) Tablets.

7200 g of granules obtained according to Example 1, A) are mixed with 60 g of magnesium stearate and 40 g of talc and compressed in a rotary pelletting machine under a pressing power of 98.1 to 196.2 N/mm² to give tablets of 730 mg weight. This gives tablets having the following properties:

| Tablet properties: | | Composition: | |
|---|---|---|---|
| Oblong tablet | | Extract substances | 400 mg |
| Length | 18 mm | Polyplasdone | 150 mg |
| Width | 7 mm | Avicel PH 101 | 150 mg |
| Thickness | 6.7 mm | Kollidon K 25 | 20 mg |
| Breaking strength | 180 N | Talc | 4 mg |
| Attrition (4 min) | 0.1% | Mg stearate | 6 mg |
| Disintegration | 10 minutes | | |

### Example 4:

### 4) Results were obtained from clinical trials using the deproteinised calf blood preparation Actovegin®.

The objective was in a randomized, controlled clinical trial to evaluate the efficacy and safety of sequential treatment using intravenous (IV) infusions of Actovegin® (2000 mg) once daily for 20 days followed by oral administration (1800 mg/day) for 140 days in patients with diabetic polyneuropathy (DPN).

### Overall conclusion of clinical trial study

The overall conclusion of the clinical study was that sequential IV and oral Actovegin® treatment over 160 days improved neuropathic symptoms, VPT, sensory function, and quality of life in type 2 diabetes patients with diabetic polyneuropathy (DPN). The results are presented in the following.

### Overall clinical trial study protocol

The following examples are based upon the results of a study with the following study protocol for the clinical trial. The study protocol was a multi-centre, double-blind, placebo-controlled, randomised, parallel group clinical trial designed to evaluate efficacy and safety of deproteinised calf blood, Actovegin®, in diabetic type 2 patients with symptomatic diabetic peripheral polyneuropathy (DPN). In total 26 centres in Russia, Ukraine, Kazakhstan have been involved and the total number of randomised patients was initially 569.

In this multicenter, randomized, double-blind trial, in total 567 patients with type 2 diabetes received IV infusions of Actovegin® (2000 mg/day) (n=281) or placebo (n=286) once daily for 20 days followed by three tablets of Actovegin® (1800 mg/day) or placebo three times daily for 140 days. Total Symptom Score (TSS) of the lower limbs and vibration perception threshold (VPT) were used as co-primary outcome measures, computed as the area under the curve (AUC) from repeated scores and divided by duration of exposure. Secondary endpoints included individual TSS symptoms, Neuropathy Impairment Score of the Lower Limbs (NIS-LL), and quality of life (SF-36).

The overall results as presented in the following are: TSS was significantly improved during Actovegin® treatment compared with placebo as assessed by AUC (-0.56 points, 95% CI: -0.85, -0.27; p=0.0003) and from baseline to 160 days (-0.86 points, 95% CI: -1.22, -0.50; p<0.0001). VPT (5 sites per foot) decreased by 3% more with Actovegin® than placebo as assessed by AUC (95% CI: 0%, 6%; p=0.084) and by 5% more after 160 days (95% CI: 1%, 9%; p=0.017). NIS-LL sensory function as assessed by AUC was significantly improved with Actovegin® versus placebo after 160 days (-0.25, 95% CI: 0.46, -0.04; p=0.021), as was the SF-36 mental health domain. There were no differences in the incidence of adverse events between the groups.

### Clinical trial study protocol in detail

Patients were followed for approximately 6 months from the screening visit to the end of the oral treatment period with efficacy assessments at screening, at every 5th infusion visit and every 4 weeks during the oral treatment period. Adverse events (AEs) were assessed at all visits. Approval was obtained from local Ethics Committees and all patients provided written informed consent. After a maximum screening period of 5 days, a total of 569 type 2 diabetes patients with symptomatic diabetic peripheral polyneuropathy (DPN) were randomly assigned via an Interactive Voice Response Service (IVRS) to treatment with either Actovegin® (Nycomed Austria GmbH) or placebo. To homogenize the study population, the randomization procedure was stratified according to site and the presence or absence of insulin treatment. Treatment consisted of 20 once daily IV infusions (Actovegin® 20% with 8 mg/ml or placebo in 250 ml sodium chloride 0.9%, infusion rate: 2 ml/min) for 20-36 days, followed by three tablets (200 mg Actovegin® per coated tablet or placebo) three times daily for 140 days, with a permitted variation of 125-155 days. In the intention-to-treat (ITT) population, the median (range) periods of IV and oral treatment were 25 (1-38) days and 146 (17-169) days for Actovegin® and 25 (1-37) days for placebo, respectively. All bottles containing solution for infusion (active and placebo) were identical and had a non-transparent plastic cover while tubes for infusion were manufactured in colored plastic material. Before blinding, i.e. before application of the plastic cover, the bottles were stored for ≥3 months and the solution checked visually for foreign bodies. The coated tablets (active and placebo) were identical in size and appearance. Inclusion criteria were: age between 18 and 65 years; type 2 diabetes according to the American Diabetes Association criteria (American Diabetes Association, Care 32, Suppl 1: S62-S67, 2009); evidence of symptomatic DPN, i.e. Total Symptom Score (TSS) ≥6 and Neuropathy Impairment Score of the Lower Limbs (NIS-LL) ≥2; vibration perception threshold (VPT) ≤30 volts; palpable pulses of posterior tibial artery and dorsal artery of foot; HbA1c<10%; patient able to meet the center visits over the trial period; stable dose of tricyclic antidepressants, anticonvulsants, mexiletine or neuroleptics in patients receiving these drugs for neuropathic pain within the last month; acceptable contraceptive method (hormonal pills, patches, implants, injections or intrauterine device) in female patients of childbearing potential; and a negative pregnancy test before 1st dose of trial medication. Exclusion criteria included known allergy to Actovegin® or similar preparations; asymmetrical neuropathy of the trunk or proximal lower limbs; foot ulcer or infection; severe cardiac failure, pulmonary edema, oliguria, anuria, or generalised edema; polyneuropathy due to causes other than diabetes; hospitalization due to DPN within the last month; prior use of medications such as isoniazid, nitrofurantoin, vincristine and phenytoin; use of cerebrolysin, α-lipoic acid, opiates, transcutaneous electrical nerve stimulation or acupuncture within the last month; mental, psychiatric or other conditions that may have compromised data collection and understanding of written and verbal information given in the trial; present and/or previous chronic alcohol abuse; and serum creatinine >120 Nmol/l.

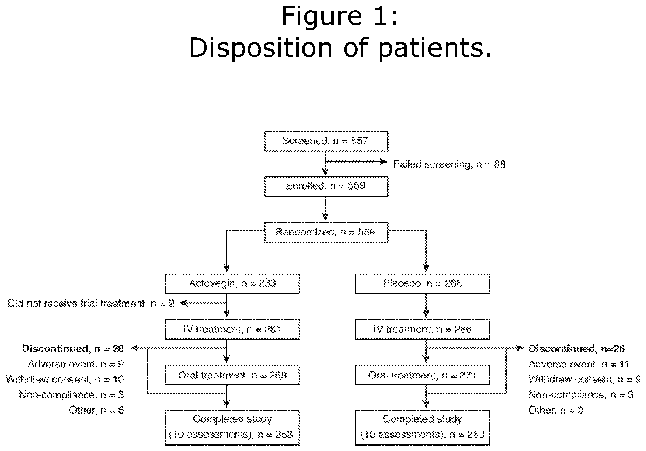

Clinical trial study results in detail:
In the following the description of the disposition of the patients, their demographics, baseline characteristics in relation to their disease is disclosed. A total of 661 patients were screened and 569 patients were randomized, 567 of whom were exposed to the study medication (ITT population), and 513 of whom completed all assessments in the study, giving a drop-out rate of 9.8%. The per protocol (PP) population consisted of 506 patients. The flow of the patients through the trial is shown in the flow diagram in figure 1. The overall demographic and clinical characteristics of the patients are shown in Table 1 followed by other tables disclosing further details of the demographic and clinical characteristics of the patients.

Safety parameters were:
Physical examination and assessment of vital signs and safety laboratory parameters were performed at screening and after the IV and oral treatment periods. Fasting blood glucose was measured at the same time intervals as the TSS. HbA1c was measured at screening, after the infusion period and after 2 and 5 months of oral treatment.

**Table 1: Demographic, laboratory, and efficacy parameters in the intention-to-treat population at baseline.**

| | Actovegin® (n=281) | Placebo (n=286) |
|---|---|---|
| Age (years) | 55.7 ± 6.4 (31-65) | 55.6 ± 6.3 (29-65) |
| Sex (% male) | 31 | 27 |
| Race (Caucasian/Mongolian) (%) | 95/5 | 93/7 |
| BMI (kg/m2) | 30.6 ± 5.5 (19.5-55.8) | 30.7 ± 4.8 (18.7-49.5) |
| Height (cm) * | 166.8 (149-190) | 166.4 (150-189) |
| Weight (kg) * | 85.2 (45-170) | 84.9 (51-146) |
| Systolic blood pressure (mmHg) | 134.6 ± 12.5 (100-180) | 135.2 ± 12.7 (105-176) |
| Diastolic blood pressure (mmHg) | 81.1 ± 7.3 (60-104) | 81.6 ± 7.4 (60-104) |
| Heart rate (bpm) | 74.1 ± 6.4 (55-98) | 74.8 ± 6.0 (52-94) |
| Smoker (%) | 10 | 15 |
| Alcohol drinker (%) | 58 | 53 |
| Insulin treatment (%) | 41 | 41 |
| Duration of diabetes (years) | 8.4 ± 6.4 (0-35) | 7.9 ± 6.7 (0-36) |
| Duration of neuropathy (years) | 2.9 ± 3.0 (0-18) | 2.5 ± 2.8 (0-20) |
| Retinopathy (%) | 26 | 19 |
| Nephropathy (%) | 5 | 4 |
| Cardiac disorders (%) | 41 | 33 |
| Peripheral artery disease (%) | 11 | 10 |
| Hypertension (%) | 79 | 81 |
| HbAlc (%) | 7.9 ± 1.5 (3.5-11.7) | 7.7 ± 1.5 (2.9-9.9) |
| Fasting blood glucose (mmol/L) | 8.4 ± 2.2 (3.5-15.5) | 8.3 ± 2.3 (4.1-17.2) |
| TSS | 8.3 ± 1.7 (6.0-14.6) | 8.4 ± 1.6 (6.0-14.0) |
| VPT (volts) | 19.7 ± 6.3 (7.2-30.0) | 20.0 ± 5.8 (7.5-30.2) |
| NIS-LL | 8.4 ± 6.5 (2.0-4 1.0) | 8.8 ± 7.3 (0.0-54.0) |
| SF-36, physical health | 39.8 ± 7.7 (17.2-61.2) | 39.9 ± 7.5 (15.7-59.7) |
| SF-36, mental health | 39.8 ± 11.(7.9-63.8) | 39.9 ± 10.3(12.4-67.1) |
| - Foot ulcerations | 6 | 2 |
| - Foot infections | 0 | 6 |
| Other | 1 | 1 |
| | | |
| Concomitant illnesses (SOC, >20%) | | |
| Total | 98% | 96% |
| Vascular disorders | 84% | 85% |
| - Eye disorders | 38% | 39% |
| - Hepatobiliary disorders | 35% | 29% |
| - Metabol / nutrit. disorders | 24% | 23% |
| - Nervous system disorders | 28% | 18% |
| - Gastrointestinal disorders | 24% | 20% |
| Mean changes from baseline to end of treatment are presented above. Thus, data represent mean ±SD unless otherwise indicated. | | |
| Exploratory analysis of individual TSS symptoms averaged over treatment duration (i.e. TSS_{average} or TSS_{AUC} is indicated in parenthesis for a number of values. | | |

**Disposition of patients**

| Trial treatment | Actovegin® | Placebo |
|---|---|---|
| Randomised to treatment | 283 | 286 |
| Received infusions | 281 | 286 |
| Received tablets | 268 | 271 |
| Discontinued | 30 | 26 |
| - Aes | 9 | 11 |
| - Consent withdrawn | 11 | 9 |
| - Non-compliance | 3 | 3 |
| - Other | 7 | 3 |
| ITT analysis set | 281 | 286 |
| PP analysis set | 250 | 256 |

As a sign of homogeneity, no clinically relevant baseline differences between the groups were noted for any of the listed parameters. The TSS, averaged over the time course of the trial, was by 0.56 points lower among patients in the Actovegin® group compared with the placebo group (p=_{0.0003}, 95% CI: 0.27; 0.85). When analyzed from baseline to 160 days, TSS improved by 0.86 points on Actovegin® compared with placebo (p<0.001; 95% CI: 0.50;1.22,). VPT decreased by 3% more in the Actovegin® group compared with the placebo group (p=0.08, 95% CI: 0%, 6%) when averaged over the course of the trial, and by 5% more after 160 days (p=0.017, 95% CI: 1%,9%).
The mean effect of Actovegin® upon TSS scores varied across centers, from -2.93 (95% CI: -4.27, -1.60) to 1.19 points (95% CI: -0.67, 3.04), with evidence of a treatment-by-center interaction (p<0.001). The effect of Actovegin® on VPT scores also varied across centers, from a reduction of 21% (95% CI: 9%; 31%) to an increase of 11% (95% CI: -9%; 35%), with evidence of a treatment-by-center interaction (p=0.02). No differences in the primary endpoints were noted between patients with and without insulin treatment. Furthermore, there was no statistically significant interaction of the treatment effect with smoking or drinking habits (data not shown).

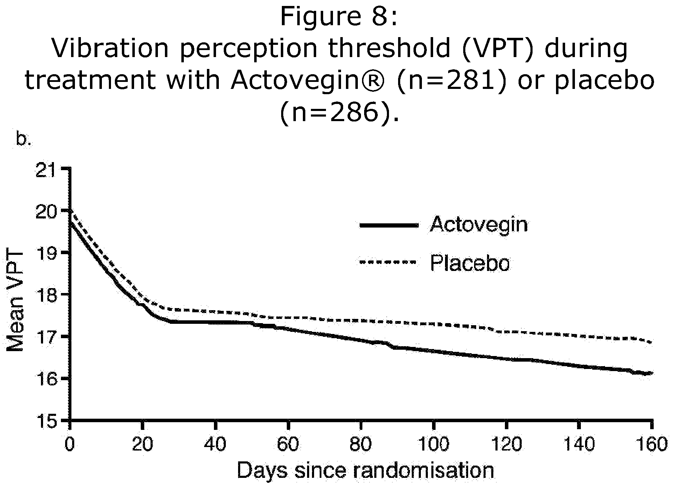

The mean values of TSS and VPT during the IV and oral treatment phases of the trial are illustrated in figure 4 and 8. A relatively high placebo effect in TSS was observed. The response rate after 160 days, if defined as a clinically meaningful reduction in TSS of ≥50%, was 73% in the Actovegin® group and 61% in the placebo group. The changes in the individual outcome measures from baseline to end of treatment in the ITT population are shown in table 2 below.

**Table 2: Changes in the individual outcome measures from baseline to end of treatment in the ITT population treated with Actovegin® (n=276) or placebo (n=280).**

| | Actovegin® (n=276) | Placebo (n=280) | Difference (95% CI) | P-value |
|---|---|---|---|---|
| TSS | -5.5 ± 2.6 | -5.5 ± 2.9 | -0.86 (-1.22, - 0.50) | <0.0001 |
| Lancinating pain | -5.5 ± 1.2 | -5.5 ± 1.2 | -0.20 (-0.32, - 0.08) | 0.002 |
| Burning pain | -5.5 ± 1.1 | -5.5 ± 1.2 | -0.26 (-0.38, - 0.14) | <0.0001 |
| Paresthesia | -5.5 ± 1.1 | -5.5 ± 1.1 | -0.21 (-0.33, - 0.09) | 0.0007 |
| Numbness | -5.5 ± 1.1 | -5.5 ± 1.1 | -0.24 (-0.38, - 0.10) | 0.0010 |
| VPT (volts) | -3.6 ± 4.5 | -2.9 ± 4.7 | -5% (-9%, - 1%) | 0.017 |
| NIS-LL | -3.9 ± 4.7 | -3.7 ± 5.0 | -0.48 (-1.01, 0.06) | 0.080 |
| NIS-LL sensory function | -2.1 ± 2.1 | -1.7 ± 2.1 | -0.38 (-0.64, - 0.12)) | 0.005 |
| NIS-LL reflexes | -0.5 ± 1.3 | -0.6 ± 1.3 | -0.05 (-0.22, 0.12) | 0.571 |
| NIS-LL muscle strength | -1.3 ± 3.1 | -1.4 ± 3.3 | -0.06 (-0.38, 0.26) | 0.731 |
| SF-36, physical health | 4.4 ± 7.0 | 3.6 ± 7.1 | 0.91 (-0.18, 2.00) | 0.101 |
| SF-36, mental health | 5.5 ± 10.6 | 3.8 ± 10.1 | 1.53 (0.17, 2.88) | 0.027 |
| Data are means ±SD, unless otherwise indicated. | | | | |
| The first two columns represent raw within group difference over time. The last two columns represent model-adjusted between group differences in the development over time. | | | | |
| TSS = Total Symptom Score, VPT = vibration perception threshold, NIS-LL = Neuropathy Impairment | | | | |
| Score of the lower limbs. | | | | |

The TSS and its individual neuropathic symptoms as well as VPT were reduced significantly more after 160 days with Actovegin® treatment than with placebo (all p<0.05). NIS-LL tended to improve with Actovegin® compared with placebo after 160 days (p=0.08) because of significantly improved sensory nerve function (p=0.005) but not muscle strength (p=0.731) or muscle reflexes (p=0.571). The mental health domain of SF-36 was significantly improved after 160 days of Actovegin® treatment compared with placebo (p=0.027), whereas changes in the physical health domain were not significantly different between groups (p=0.101). After 160 days, HbA1c decreased by -0.15 ± 1.48% in the Actovegin® group and increased by 0.10 ± 1.65% in the placebo group (p=0.04 between groups). Fasting blood glucose decreased from baseline to end of study by -0.40 ± 2.66 mmol/L in the Actovegin® group and by -0.18 ± 2.51 mmol/L in the placebo group (p=0.19 between groups).

Safety analysis did not reveal any relevant differences in treatment-emergent AEs (TEAEs) or serious AEs (SAEs) between groups. There were 186 TEAEs in 92 patients in the Actovegin® group, while 198 TEAEs occurred in 100 patients in the placebo group. Of these TEAEs, 41 (22%) and 35 (18%) were considered possibly or probably related to Actovegin® and placebo, respectively. Ten SAEs were reported in 7 patients during Actovegin® treatment, while 11 SAEs occurred in 10 patients treated with placebo. The most frequent SAEs were cardiac disorders (7 events in 6 patients) and infections (5 events in 5 patients). No deaths occurred during the study.

### Clinical trial study conclusion in detail

The results of this multicenter, randomized, controlled clinical trial showed that treatment of symptomatic DPN with IV infusions of Actovegin® (2000 mg) once daily for 20 days followed by oral administration (1800 mg/day) for 140 days improved neuropathic symptoms as scored by the TSS, VPT on both feet, the sensory nerve function component of the NIS-LL, and quality of life as evidenced by the mental health domain of the SF-36. This trial also confirmed the favorable safety profile of Actovegin, which has been demonstrated in previous controlled clinical trials and during almost 50 years of post-marketing experience.

The magnitude of the treatment effect observed in this trial deserves comment. A consensus panel previously suggested that a clinically meaningful difference between active treatment and placebo for changes in positive sensory symptoms from baseline is 0.834 points on a 10-point scale and one point on a scale similar to the TSS, if an average of several symptom descriptors is used (Apfel SC et al., J Neurol Sci 189: 3-5, 2001). As the mean difference for the changes in TSS between Actovegin® and placebo after 160 days was 0.86 points, we believe that the drug exerted a clinically meaningful effect on the main neuropathic symptoms. However, an essential prerequisite for a disease-modifying drug treatment to be effective is a favourable impact on the natural progression of DPN, which is primarily driven by the sensory neuropathic deficits (impairments) rather than symptoms. In this trial, improvement was observed for both neuropathic symptoms (TSS symptoms) and sensory deficits (VPT), NIS-LL sensory component. Elevated VPT is an independent risk factor for the development of diabetic foot ulcers. In a 1-year prospective multicenter study, the hazard of the first foot ulcer increased by 5.6 % with each volt increase in VPT at baseline (Abbott CA et al., Diabetes Care 21: 1071-1075, 1998). In the present trial the improvement in VPT from baseline to the end of the study was significantly larger in the Actovegin® group compared with the placebo group (between-group difference: 5%, 95% CI: 1%, 9%, p=0.017). With a baseline mean of approximately 20 volts, the implication is that VPT improved by one volt more in the Actovegin® group than the placebo group. Thus, the observed effect of Actovegin® on VPT appears to reflect a clinically relevant improvement.

This study has several limitations. First, perhaps not surprisingly given that a total of 26 centers from three countries participated in this trial, a center-treatment interaction effect was noted. Although intensive training was carried out in order to standardize all relevant procedures, inter-center variations were to be expected. However, it was reassuring that the observed treatment effects persisted after appropriate adjustment for center. Second, treatment with Actovegin® was associated with a slight improvement in HbA1c levels, resulting in a mean difference versus placebo of 0.25%. When adjusting for the changes in HbA1c, the treatment effect on the TSS decreased from 0.86 to 0.83 points. Thus, although the effect of Actovegin® on HbA1c was statistically significant, as well as potentially beneficial, we consider the effect size unlikely to introduce bias toward the observed favorable effect of Actovegin® on TSS. Third, no objective peripheral nerve function tests such as nerve conduction studies were used which could have been more sensitive to a treatment effect with Actovegin® than VPT as a psychophysical measure. Forth, a relatively high placebo effect on the TSS was noted. In the placebo group, the response rate, if defined as a clinically meaningful reduction in TSS of ≥50%, reached 61%. Despite such a high placebo effect, the corresponding response rate in the Actovegin® group of 73% may be considered clinically relevant, as the relative advantage of Actovegin® versus placebo was around 20%. A recent metaanalysis showed that the placebo effect does not reach a plateau even after 19 weeks of treatment but tends to continue (Quessy SN and Rowbotham MC, Pain 138: 479-483, 2008). Thus, sustained improvement of symptoms such as pain in the placebo group with increasing duration of the trial renders it difficult to show superiority of the active drug over placebo.

The mechanisms by which Actovegin® exerts its effect on neuropathic symptoms is not clear, but have been described in the following references:
Kuninaka T et al. J Cell Physiol 146: 148-155, 1991; Obermaier-Kusser B et al., Biochem J 261: 699-705, 1989; Jacob S et al. Arzneimittelforschung 46: 269-272, 1996; Kuninaka T et al. J Cell Physiol 146: 148-155, 1991; De Groot et al., Res Comm Chem Path Pharm 68: 125-128, 1990; Cameron NE et al., Diabetologia 44: 1973-1988, 2001; Machicao F et al., Biochem J 266: 909-916, 1990; Gottschalk WK and Jarret L, Arch Biochem Biophys 261: 175-185, 1988; Fox JA et al., Proc Natl Acad Sci USA 84: 2663-2667, 1987. Without being bound by any theory, it is contemplated that Actovegin® exerts an insulin-like effect leading to enhancement of glucose utilization with a direct impact on the cellular metabolism and energy balance in distinct cellular systems.

In conclusion, treatment with Actovegin® IV for 20 days and subsequent oral treatment for 140 days was safe and effective in improving neuropathic symptoms, VPT, sensory nerve function, and mental health in type 2 diabetes patients with symptomatic polyneuropathy (DPN). The mechanisms by which Actovegin® exerts these favourable effects on nerve function remain to be established.

The two co-primary end points were the TSS and VPT. TSS was assessed at screening, after 5, 10, 15, and 20 infusions, and every 4 weeks (± 5 days) during the oral treatment period.

In the following, further details of the Total Symptom Score (TSS) results are presented and the scheme for evaluating TSS is also outlined in figure 3.

**Summary of the TSS scores.**

| | Actovegin® | | | | Placebo | | | |
|---|---|---|---|---|---|---|---|---|
| | N | Mean | Min | Max | N | Mean | Min | Max |
| Baseline | 281 | 8.3 | 6.0 | 14.6 | 286 | 8.4 | 6.0 | 14.0 |
| End of infusions | 276 | 4.4 | 0.0 | 10.7 | 280 | 4.9 | 0.0 | 11.7 |
| End of trial | 276 | 2.8 | 0.0 | 9.7 | 280 | 3.7 | 0.0 | 11.0 |

Mean TSS profile is presented in figure 4.

**Primary analysis of TSSaverage (N=556).**

| | | 95% CI | | |
|---|---|---|---|---|
| | Mean | Lower | Upper | P-value |
| Actovegin® | 3.77 | 3.52 | 4.01 | |
| Placebo | 4.33 | 4.08 | 4.57 | |
| Actovegin® - Placebo | -0.56 | -0.85 | -0.27 | 0,0003 |

**Summary of the change in TSS at end of infusions and end of trial.**

| | Actovegin® | | | | Placebo | | | |
|---|---|---|---|---|---|---|---|---|
| | N | Mean | Min | Max | N | Mean | Min | Max |
| End of infusions | 276 | -3.8 | -11.6 | 2.0 | 280 | -3.6 | -12.3 | 1.7 |
| End of trial | 276 | -5.5 | -13.6 | 0.3 | 280 | -4.7 | -13.0 | 2.3 |

**Exploratory analysis of TSS (N=556) mean change from baseline.**

| | | 95% CI | | |
|---|---|---|---|---|
| | Mean Treatment Estimate | Lower | Upper | P-value |
| End of trial - Baseline | -0,86 | -1.22 | -0,50 | < .0001 |
| End of Infusion - Baseline | -0,34 | -0,66 | -0,02 | 0.0380 |

**Exploratory analysis of TSSaverage treatment by smoking/drinking interaction.**

| | | 95% CI | | |
|---|---|---|---|---|
| | Actovegin® / Placebo | Lower | Upper | P-value |
| Treatment effect in drinkers / non-drinkers | | | | |
| Non-drinkers (n=458) | -.645 | -0.96 | -0.33 | |
| Drinkers (n=98) | -.147 | -0.84 | 0.55 | |
| Treatment x Alcohol interaction | | | | 0.205 |
| | | | | |
| Treatment effect in smokers / non-smokers | | | | |
| Non-smokers (n=486) | -.614 | -0.92 | -0.30 | |
| Smokers (n=70) | -.218 | -1.07 | 0.63 | |
| Treatment x Smoking interaction | | | | 0.395 |

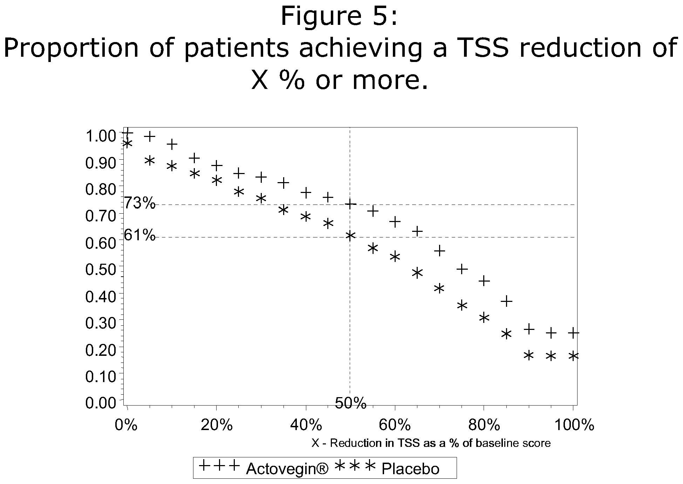

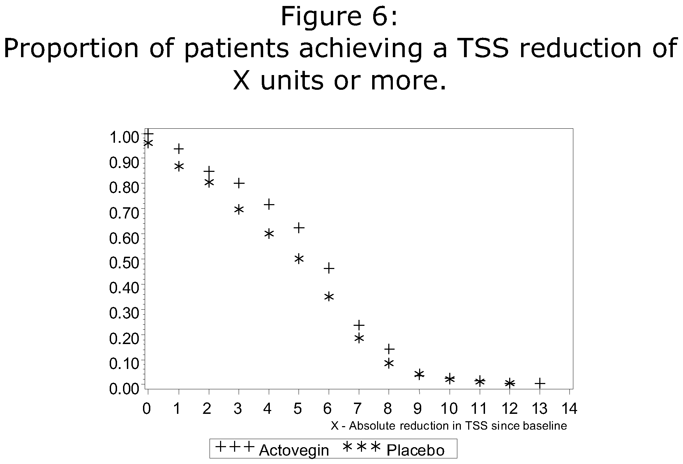

Figure 5 shows the proportion of patients who achieved a TSS reduction of X % or more whereas Figure 6 shows the proportion of patients who achieved a TSS reduction of X units or more.

Exploratory analysis of the individual TSS averaged over duration of exposure (N=556). Thus data in this table correspond to TSS_{AUC}. The values presented in Table 2 correspond to mean TSS results.

| | | | 95% CI | | |
|---|---|---|---|---|---|
| Symptom | Effect | Mean | Lower | Upper | P-value |
| Lancinating pain | Actovegin® | 0.68 | 0.60 | 0.76 | |
| | Placebo | 0.84 | 0.77 | 0.92 | |
| | Actovegin® - Placebo | -0.16 | -0.25 | -0.07 | 0.0005 |
| | | | | | |
| Burning pain | Actovegin® | 0.81 | 0.72 | 0.89 | |
| | Placebo | 0.99 | 0.91 | 1.07 | |
| | Actovegin® - Placebo | -0.18 | -0.28 | -0.09 | 0.0002 |
| Prickling | Actovegin® | 0.95 | 0.87 | 1.03 | |
| | Placebo | 1.07 | 0.99 | 1.15 | |
| | Actovegin® - Placebo | -0.12 | -0.22 | -0.02 | 0.0146 |
| Numbness | Actovegin® | 1.29 | 1.19 | 1.38 | |
| | Placebo | 1.41 | 1.32 | 1.51 | |
| | Actovegin® - Placebo | -0.12 | -0.24 | -0.01 | 0.0268 |

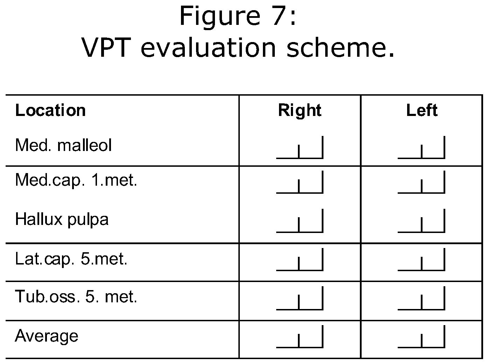

In the following examples the Vibration Perception Threshold (VPT) results are presented in further detail. A scheme for evaluating VTP is shown in figure 7. VPT was assessed at screening, after 5, 10, 15, and 20 infusions, and every 4 weeks (± 5 days) during said oral treatment period.

**Summary of the VPT scores.**

| | Actovegin® | | | | Placebo | | | |
|---|---|---|---|---|---|---|---|---|
| | N | Mean | Min | Max | N | Mean | Min | Max |
| Baseline | 281 | 19.7 | 7.2 | 30.0 | 286 | 20.0 | 7.5 | 30.2 |
| End of infusions | 276 | 17.4 | 5.0 | 33.5 | 280 | 17.9 | 5.7 | 29.9 |
| End of trial | 276 | 16.2 | 3.9 | 30.3 | 280 | 17.2 | 4.2 | 31.6 |

The mean VPT profiles are presented in figure 8.

**Primary analysis of VPTaverage* (N=556).**

| | | 95% CI | | |
|---|---|---|---|---|
| | Geo. Mean | Lower | Upper | P-value |
| Actovegin® | 16.24 | 15.83 | 16.66 | |
| Placebo | 16.68 | 16.26 | 17.11 | |
| Actovegin® / Placebo | 0.97 | 0.94 | 1.00 | 0.084 |

| | | | | |
|---|---|---|---|---|
| * = log transformed in the analysis | | | | |

**Summary of the change in VPT at end of infusions and end of trial.**

| | Actovegin® | | | | Placebo | | | |
|---|---|---|---|---|---|---|---|---|
| | N | Mean | Min | Max | N | Mean | Min | Max |
| End of infusions | 276 | -2.4 | -15.8 | 6.1 | 280 | -2.2 | -12.3 | 6.2 |
| End of trial | 276 | -3.6 | -20.6 | 9.5 | 280 | -2.9 | -20.2 | 13.4 |

**Exploratory analysis of VPT* (N=556) mean change from baseline.**

| | | 95% CI | | |
|---|---|---|---|---|
| | Actovegin® / Placebo | Lower | Upper | P-value |
| End of trial - Baseline (N=556) | 0.95 | 0.91 | 0.99 | 0.017 |
| End of Infusion - Baseline (N=556) | 0.98 | 0.96 | 1.01 | 0.232 |

| | | | | |
|---|---|---|---|---|
| * = log transformed in the analysis | | | | |

**Exploratory analysis of VPTaverage* treatment by smoking/drinking interaction, ITT**

| | | 95% CI | | |
|---|---|---|---|---|
| | Actovegin® / Placebo | Lower | Upper | P-value |
| Treatment effect in drinkers / non-drinkers | | | | |
| Non-drinkers (n=458) | 0.97 | 0.94 | 1.00 | |
| Drinkers (n=98) | 1.00 | 0.93 | 1.07 | |
| Treatment x Alcohol interaction | | | | 0.489 |
| | | | | |
| Treatment effect in smokers / non-smokers | | | | |
| Non-smokers (n=486) | 0.97 | 0.94 | 1.01 | |
| Smokers (n=70) | 0.98 | 0.90 | 1.07 | |
| Treatment x Smoking interaction | | | | 0.878 |

| | | | | |
|---|---|---|---|---|
| * = log transformed in the analysis | | | | |

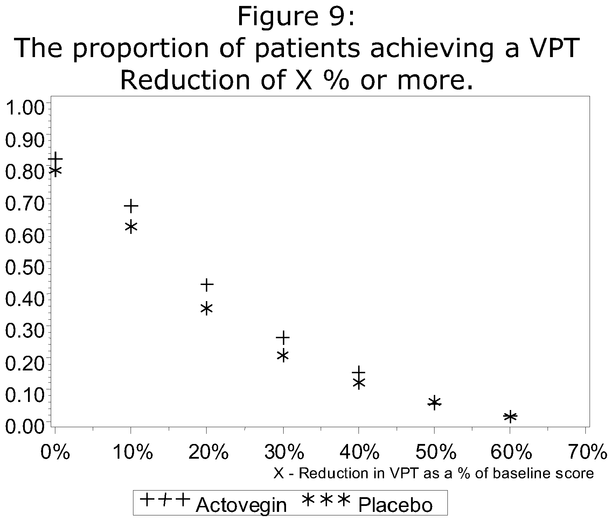

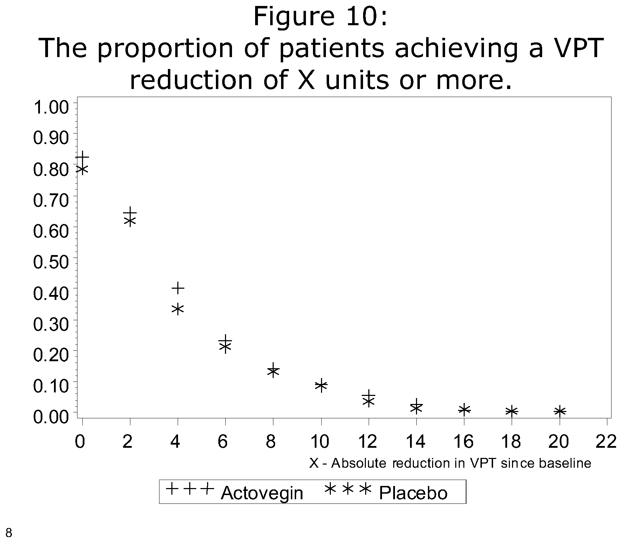

Figure 9 shows the proportion of patients who achieved a VPT Reduction of X % or more and figure 10 shows the proportion of patients achieving a VPT Reduction of X units or more.

**Exploratory analysis of the Hallux Pulpa VPT* averaged over duration of exposure (N=556).**

| | | 95% CI | | |
|---|---|---|---|---|
| | Geo. Mean | Lower | Upper | P-value |
| Actovegin® | 16.01 | 15.54 | 16.51 | |
| Placebo | 16.58 | 16.08 | 17.09 | |
| Actovegin® / Placebo | 0.97 | 0.93 | 1.00 | 0.059 |

| | | | | |
|---|---|---|---|---|
| * = log transformed | | | | |

In the following the secondary outcome measure is disclosed:
The NIS-LL was assessed on the same days as the primary endpoints and was computed as the sum score of a standard group of examinations of muscle strength (0 = normal to 4 = paralyzed), reflexes (0 = normal to 2 = absent with reinforcement), and touch-pressure, vibration, joint position and motion, and pinprick sensation (0 = normal to 2 = absent for each modality) of the great toe and was scored for both sides of the body (Dyck PJ et al., Neurology 42:1164-1170, 1992). Thus, NIS-LL was assessed at screening, after 5, 10, 15, and 20 infusions, and every 4 weeks (± 5 days) during said oral treatment period. All participating centers were trained by a senior neurologist (IS) to adequately perform the NIS-LL. Quality of life was assessed by the SF-36 questionnaire, second version, validated in local languages (McHorney CA et al., Med Care 32: 40-66, 1994) and was completed by patients at randomization and after the IV and oral treatment periods. Additional exploratory analyses included the scores of the four individual TSS symptoms and the three individual components of the NIS-LL. In addition, the effects of alcohol use (categorized as never, monthly or less, 2 to 4 times a month, 2 to 3 times a week, and 4 or more times a week) and smoking habits on treatment were assessed.

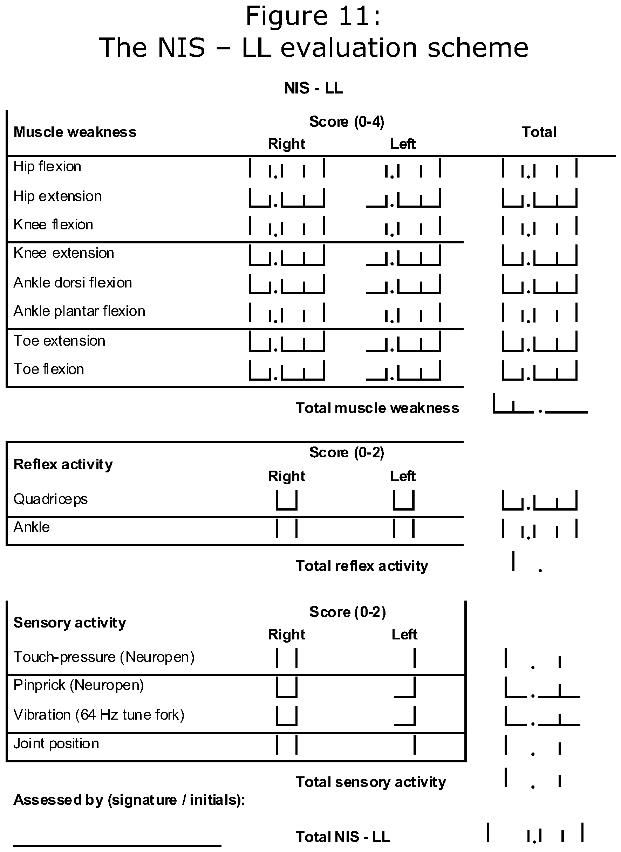

In the following, results obtained with measuring the Neuropathy Impairment Score of the lower limbs (NIS-LL) are presented in further detail along with the scheme for evaluating NIS-LL. Figure 11 shows the NIS - LL evaluation scheme.

**Summary of the NIS-LL scores at baseline, end of infusions and end of trial, ITT**

| | Actovegin® | | | | Placebo | | | |
|---|---|---|---|---|---|---|---|---|
| | N | Mean | Min | Max | N | Mean | Min | Max |
| Baseline | 281 | 8.4 | 2.0 | 41.0 | 286 | 8.8 | 0.0 | 54.0 |
| End of Infusions | 276 | 5.8 | 0.0 | 51.0 | 280 | 6.4 | 0.0 | 52.0 |
| End of Trial | 276 | 4.4 | 0.0 | 50.0 | 280 | 5.2 | 0.0 | 42.0 |

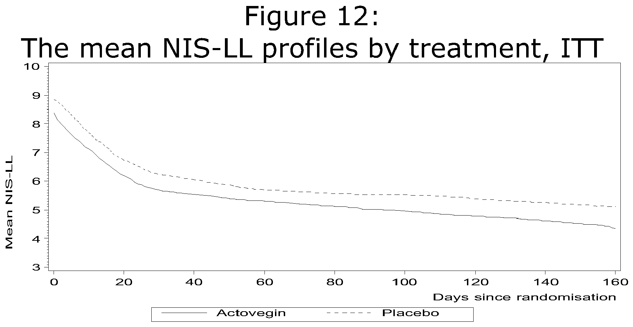

Figure 12 shows the mean NIS-LL profiles by treatment, ITT.

**Secondary endpoint analysis of NIS-LLaverage (N=556)**

| | | 95% CI | | |
|---|---|---|---|---|
| | Mean | Lower | Upper | P-value |
| Actovegin® | 5.80 | 5.42 | 6.18 | |
| Placebo | 6.06 | 5.68 | 6.45 | |
| Actovegin® / Placebo | -0.26 | -0.72 | 0.19 | 0.253 |

**Summary of the change in NIS-LL at end of infusions and end of trial**

| | Actovegin® | | | | Placebo | | | |
|---|---|---|---|---|---|---|---|---|
| | N | Mean | Min | Max | N | Mean | Min | Max |
| End of infusions | 276 | -2.5 | -29.0 | 11.0 | 280 | -2.5 | -22.0 | 5.0 |
| End of trial | 276 | -3.9 | -34.0 | 10.0 | 280 | -3.7 | -32.0 | 4.0 |

**Exploratory analysis of NIS-LL - mean change from baseline (N=556).**

| | | 95% CI | | |
|---|---|---|---|---|
| | Mean | Lower | Upper | P-value |
| Baseline - End of trial | -0.48 | -1.01 | 0.06 | 0.008 |
| Baseline - End of Infusion | -0.21 | -0.67 | 0.26 | 0.387 |

**Exploratory NIS-LL analysis of muscle weakness, reflex and sensory activity separately - mean change from baseline.**

| | | 95% CI | | |
|---|---|---|---|---|
| | Mean | Lower | Upper | P-value |
| Muscle weakness: Baseline - End of Trial | -0.06 | -0.38 | 0.26 | 0.731 |
| Reflex activity: Baseline - End of Trial | -0.05 | -0.22 | 0.12 | 0.571 |
| Sensory activity: Baseline - End of Trial | -0.38 | -0.64 | -0.12 | 0.005 |

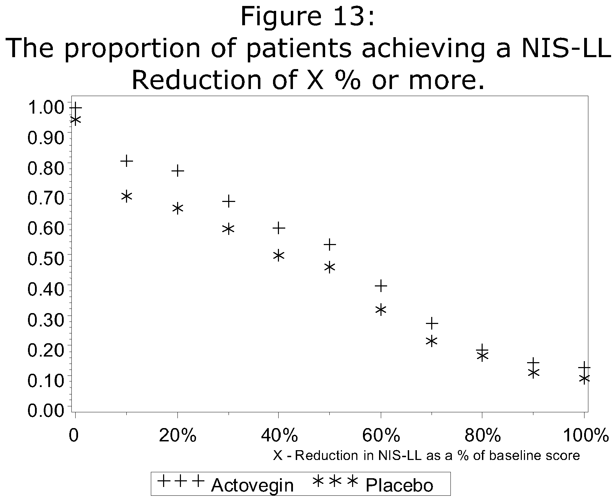

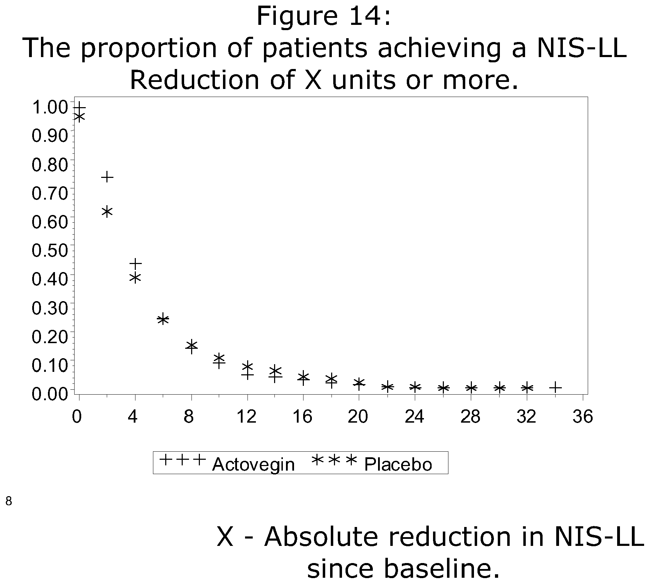

Figure 13 shows the proportion of patients who achieved a NIS-LL Reduction of X % or more whereas figure 14 shows the proportion of patients who achieved a NIS-LL Reduction of X units or more.

In the following, results obtained measuring the Quality of Life (QoL) are presented.

**Summary of the QoL (SF-36) scores by visit and treatment.**

| | Actovegin® | | | | Placebo | | | |
|---|---|---|---|---|---|---|---|---|
| | N | Mean | Min | Max | N | Mean | Min | Max |
| Physical Health | | | | | | | | |
| Baseline | 274 | 39.8 | 17.2 | 61.2 | 270 | 39.9 | 15.7 | 59.7 |
| Visit 21 | 255 | 42.9 | 23.8 | 61.8 | 254 | 42.1 | 25.0 | 65.3 |
| Visit 26 | 254 | 44.4 | 24.3 | 61.9 | 263 | 43.1 | 17.0 | 60.8 |

| Mental Health | | | | | | | | |
|---|---|---|---|---|---|---|---|---|
| Baseline | 274 | 39.8 | 7.9 | 63.8 | 270 | 39.9 | 12.4 | 67.1 |
| Visit 21 | 255 | 44.5 | 18.7 | 65.0 | 254 | 43.3 | 13.3 | 67.5 |
| Visit 26 | 254 | 45.3 | 15.7 | 68.7 | 263 | 43.6 | 12.6 | 67.9 |

**QoL analysis (SF-36) at end of trial (visit 26).**

| | | 95% CI | | |
|---|---|---|---|---|
| | Actovegin® ÷ Placebo | Lower | Upper | P-value |
| Physical Health | 0.91 | -0.18 | 2.00 | 0.1013 |
| Mental Health | 1.53 | 0.17 | 2.88 | 0.0271 |

In the following, the adverse effects (AE) and clinical safety are presented.

### AEs/clinical safety

A total of 384 AEs were reported (Actovegin® :186, placebo 198) from 192 patients.

The most frequent were headache (41; Actovegin® : 22, placebo: 19), hypoglycaemia (40; 21, 19), hypertension (23; 10, 13), hyperglycaemia (22; 6, 16), blood pressure increased (18; 7, 11) and respiratory tract infection (13; 8, 5) 21 SAEs were reported: 10 in 7 Actovegin® patients and 11 in 10 placebo patients.

Causality was considered possible for one SAE (Actovegin®: cardiac failure) and probable for one (placebo: hypersensitivity).

### No patients died.

In the following the statistical analysis is presented:
The two primary outcome measures (TSS and VPT) were computed as the AUC averaged over the time of exposure. The AUC calculations were performed by the trapezoidal method. Intermediate missing values were interpolated linearly in the calculations. VPT was log-transformed. If a patient dropped out prematurely, the average was calculated for the exposure period. The primary analysis included the intention-to-treat (ITT) population. To support the primary analysis, a comparison of the mean change in the individual outcome measures from baseline to end of trial in the two treatment groups was calculated. An ANCOVA with treatment, center, and insulin treatment stratum as fixed effects (VPT additionally adjusted for age) and the baseline outcome measure as a covariate was used. Based on the linear model, an F-test was used to test the effect of treatment. The mean difference between treatments was estimated with a 95% confidence interval (CI) based on the model. Since there were two primary endpoints, the Hochberg procedure was applied for multiplicity adjustment (Hochberg Y, Biometrika 75: 800-802, 1988), ensuring an overall significance level of ≤5%. As a consequence of the multiplicity adjustment, a significant result (after adjustment) for either of the primary endpoints indicated a positive study outcome for the given endpoint. Possible center interaction was explored by including an interaction term in the ANCOVA model as a sensitivity analysis. Additional supportive analyses included smoking and alcohol use as separate covariates in the ANCOVA. The power of the trial was required to be 90%. The sample size consideration was based on a two-sample t-test of the hypothesis of no mean difference between treatments. The sample size calculation was based on the TSS for which a mean of one point is considered as the minimum clinically meaningful treatment difference (Apfel SC et al., J Neurol Sci 189: 3-5, 2001). The sample size based on the TSS was set to 480 patients with an assumed standard deviation of 3.1. To compensate for possible drop-outs, the final sample size required was 550 patients. Throughout the statistical analyses, two-sided tests at a significance level of α=0.05 were used.

### References

Abbott CA et al. Diabetes Care 1998, vol. 21 (7), 1071-1075
Abbott CA et al., Diabetes Care 21: 1071-1075, 1998
American Diabetes Association, Care 32, Suppl 1: S62-S67, 2009
Apfel SC et al., J Neurol Sci 189: 3-5, 2001
Boulton AJM et al., Diabetes Care 28, 956-962, 2005
Cameron NE et al., Diabetologia 44: 1973-1988, 2001
Chalk C et al. Cochrane Database Syst Rev 4: CD004572, 2007
Coppini DV et al. J. Clinical Neuros (2001), 8(6), 520-524
Davies M et al., Diabetes Care 29: 1518-1522, 2006
De Groot et al., Res Comm Chem Path Pharm 68: 125-128, 1990
Dworkin RH et al.,Pain 132: 237-251, 2007
Dyck PJ et al., Neurology 42:1164-1170, 1992
Fox JA et al., Proc Natl Acad Sci USA 84: 2663-2667, 1987
Galer BS et al., Diabetes Res Clin Pract 47: 123-128, 2000
Gottschalk WK and Jarret L, Arch Biochem Biophys 261: 175-185, 1988
Herrmann WM et al., Z Geriatrie 5: 46-55, 1992
Hochberg Y, Biometrika 75: 800-802, 1988
Jacob S et al. Arzneimittelforschung 46: 269-272, 1996
Jansen W. and Beck E, Die Medizinske Welt, Behandlung der diabetischen Polyneuropathie - eine kontrollierte Doppelblindstudie, medwelt 1986, 37:838-841, 1987
Kanowski S et al., Pharmacopsychiat 28: 125-133, 1995
Kuninaka T et al. J Cell Physiol 146: 148-155, 1991
Luscombe F.A. Value in Health, vol. 3, suppl. 1, 2000, 15-28
Machicao F et al., Biochem J 266: 909-916, 1990
McHorney CA et al., Med Care 32: 40-66, 1994
Obermaier-Kusser B et al., Biochem J 261: 699-705, 1989
Quessy SN and Rowbotham MC, Pain 138: 479-483, 2008
Ziegler D et al, Current Diabetes Reports, vol. 7, 6, 2007.
Ziegler D. et al, Diabetes Care. 2009 Aug;32(8):1479-84
Ziegler D et al., Diabetes Care Vol. 31: 464-469, 2008
Ziegler D et al: Diabetes Care, vol 29, 11, 2006.
Ziegler D et al., Diabetes Care, Vol 22 (8), 1999, 1296-1301
Ziegler D et al., Diabetologia 38: 1425-1433, 1995
Ziegler D et al., Diabetologia, Vol 38 (12), 1995, 1425-33
RU 2 282 453
DE 1 076 888
CN 1 895 274
XP 002533167: Simeonov S et al "Therapeutic efficacy of Milgamma in patients with painful diabetic neuropathy".

## Claims

1. Use of a deproteinised calf blood preparation for the preparation of a medicament for the prevention or treatment of diabetic peripheral polyneuropathy, wherein 1000 mg or 2000 mg of the deproteinised calf blood preparation is administered intravenously daily during a period of at least 10 consecutive days and wherein the intravenous administration is followed by a period of oral administration of 200 mg, 400 mg or 600 mg of the deproteinised calf blood preparation once, twice or three times daily for a period of 120 days or even longer as a substantially continous treatment.

2. Use of a deproteinised calf blood preparation for the preparation of a medicament for the prevention or treatment of diabetic peripheral polyneuropathy according to claim 1, wherein 250 ml of deproteinised calf blood preparation having a content of active substances of 8 mg/ml is administered intravenously once daily.

3. Use of a deproteinised calf blood preparation for the preparation of a medicament for the prevention or treatment of diabetic peripheral polyneuropathy according to claim 1 or 2, wherein 400 mg of the deproteinised calf blood preparation is administered orally three times daily.

4. Use of a deproteinised calf blood preparation for the preparation of a medicament for the prevention or treatment of diabetic peripheral polyneuropathy according to any one of claims 1 to 3, wherein 600 mg of the deproteinised calf blood preparation is administered orally three times daily.

5. Use of a deproteinised calf blood preparation for the preparation of a medicament for the prevention or treatment of diabetic peripheral polyneuropathy according to any one of claims 1 to 4, wherein the deproteinised calf blood preparation is administered orally three times daily for 140 days.

6. Use of a deproteinised calf blood preparation for the preparation of a medicament for the prevention or treatment of diabetic peripheral polyneuropathy according to any one of claims 1 to 5, wherein the deproteinised calf blood preparation is administered orally three times daily for 150 days or even longer as a substantially continuous treatment.

7. A deproteinised calf blood preparation for use in the prevention or treatment of diabetic peripheral polyneuropathy wherein 1000 mg or 2000 mg of the deproteinised calf blood preparation is administered intravenously daily during a period of at least 10 consecutive days and wherein the intraveneous administration is followed by a period of oral administration of 200 mg, 400 mg or 600 mg of the deproteinised calf blood preparation once, twice or three times daily for a period of 120 days or even longer as a substantially continuous treatment.

8. A deproteinised calf blood preparation for use according to claim 7, wherein 250 ml of deproteinised calf blood preparation having a content of active substances of 8 mg/ml is administered intravenously once daily.

9. A deproteinised calf blood preparation for use according to claim 7 or 8, wherein 400 mg of the deproteinised calf blood preparation is administered orally three times daily.

10. A deprotenised calf blood preparation for use according to any one of claims 7 to 9, wherein 600 mg of the deproteinised calf blood preparation is administered orally three times daily.

11. A deprotenised calf blood preparation for use according to any one of claims 7 to 10, wherein the deproteinised calf blood preparation is administered orally three times daily for 140 days.

12. A deprotenised calf blood preparation for use according to any one of claims 7 to 11, wherein the deproteinised calf blood preparation is administered orally three times daily for 150 days or even longer as a substantially continuous treatment.

## Patentansprüche

1. Anwendung einer deproteinisierten Kalbsblutzubereitung für die Herstellung eines Medikaments für die Vorbeugung oder Behandlung von peripherer Diabetes-Polyneuropathie, wobei 1000 mg oder 2000 mg der deproteinisierten Kalbsblutzubereitung täglich intravenös über einen Zeitraums von zumindest 10 aufeinander folgenden Tagen verabreicht werden, und wobei der intravenösen Verabreichung ein Zeitraum mit oraler Verabreichung von 200 mg, 400 mg oder 600 mg der deproteinisierten Kalbsblutzubereitung einmal, zweimal oder dreimal täglich über einen Zeitraum von 120 Tagen oder sogar länger als eine im Wesentlichen dauerhafte Behandlung folgt.

2. Anwendung einer deproteinisierten Kalbsblutzubereitung für die Herstellung eines Medikaments für die Vorbeugung oder Behandlung von peripherer Diabetes-Polyneuropathie nach Anspruch 1, wobei 250 ml der deproteinisierten Kalbsblutzubereitung mit einem Wirkstoffgehalt von 8 mg/ml einmal täglich intravenös verabreicht werden.

3. Anwendung einer deproteinisierten Kalbsblutzubereitung für die Herstellung eines Medikaments für die Vorbeugung oder Behandlung von peripherer Diabetes-Polyneuropathie nach Anspruch 1 oder 2, wobei 400 mg der deproteinisierten Kalbsblutzubereitung dreimal täglich oral verabreicht werden.

4. Anwendung einer deproteinisierten Kalbsblutzubereitung für die Herstellung eines Medikaments für die Vorbeugung oder Behandlung von peripherer Diabetes-Polyneuropathie nach einem der Ansprüche 1 bis 3, wobei 600 mg der deproteinisierten Kalbsblutzubereitung dreimal täglich oral verabreicht werden.

5. Anwendung einer deproteinisierten Kalbsblutzubereitung für die Herstellung eines Medikaments für die Vorbeugung oder Behandlung von peripherer Diabetes-Polyneuropathie nach einem der Ansprüche 1 bis 4, wobei die deproteinisierte Kalbsblutzubereitung dreimal täglich über 140 Tage oral verabreicht wird.

6. Anwendung einer deproteinisierten Kalbsblutzubereitung für die Herstellung eines Medikaments für die Vorbeugung oder Behandlung von peripherer Diabetes-Polyneuropathie nach einem der Ansprüche 1 bis 5, wobei die deproteinisierte Kalbsblutzubereitung dreimal täglich über 150 Tage oder sogar länger als eine im Wesentlichen dauerhafte Behandlung oral verabreicht wird.

7. Deproteinisierte Kalbsblutzubereitung zur Anwendung bei der Vorbeugung oder Behandlung von peripherer Diabetes-Polyneuropathie, wobei 1000 mg oder 2000 mg der deproteinisierten Kalbsblutzubereitung täglich intravenös über einen Zeitraums von zumindest 10 aufeinander folgenden Tagen verabreicht werden, und wobei der intravenösen Verabreichung ein Zeitraum mit oraler Verabreichung von 200 mg, 400 mg oder 600 mg der deproteinisierten Kalbsblutzubereitung einmal, zweimal oder dreimal täglich über einen Zeitraum von 120 Tagen oder sogar länger als eine im Wesentlichen dauerhafte Behandlung folgt.

8. Deproteinisierte Kalbsblutzubereitung zur Anwendung nach Anspruch 7, wobei 250 ml der deproteinisierten Kalbsblutzubereitung mit einem Wirkstoffgehalt von 8 mg/ml einmal täglich intravenös verabreicht werden.

9. Deproteinisierte Kalbsblutzubereitung zur Anwendung nach Anspruch 7 oder 8, wobei 400 mg der deproteinisierten Kalbsblutzubereitung dreimal täglich oral verabreicht werden.

10. Deproteinisierte Kalbsblutzubereitung zur Anwendung nach einem der Ansprüche 7 bis 9, wobei 600 mg der deproteinisierten Kalbsblutzubereitung dreimal täglich oral verabreicht werden.

11. Deproteinisierte Kalbsblutzubereitung zur Anwendung nach einem der Ansprüche 7 bis 10, wobei die deproteinisierte Kalbsblutzubereitung dreimal täglich über 140 Tage oral verabreicht wird.

12. Deproteinisierte Kalbsblutzubereitung zur Anwendung nach einem der Ansprüche 7 bis 11, wobei die deproteinisierte Kalbsblutzubereitung dreimal täglich über 150 Tage oder sogar länger als eine im Wesentlichen dauerhafte Behandlung oral verabreicht wird.

## Revendications

1. Utilisation d'une préparation de sang de veau déprotéiné pour la préparation d'un médicament à utiliser dans la prévention ou le traitement de la polyneuropathie périphérique diabétique, dans laquelle 1000 mg ou 2000 mg de la préparation de sang de veau déprotéiné sont administrés quotidiennement par voie intraveineuse pendant une période d'au moins 10 jours consécutifs et dans laquelle l'administration par voie intraveineuse est suivie par une période d'administration par voie orale de 200 mg, de 400 mg ou de 600 mg de la préparation de sang de veau déprotéiné en une, deux ou trois prises quotidiennes sur une période de 120 jours voire même plus longtemps en tant que traitement sensiblement continu.

2. Utilisation d'une préparation de sang de veau déprotéiné pour la préparation d'un médicament à utiliser dans la prévention ou le traitement de la polyneuropathie périphérique diabétique selon la revendication 1, dans laquelle 250 ml de préparation de sang de veau déprotéiné ayant une teneur en substances actives de 8 mg/ml sont administrés une fois par jour par voie intraveineuse.

3. Utilisation d'une préparation de sang de veau déprotéiné pour la préparation d'un médicament à utiliser dans la prévention ou le traitement de la polyneuropathie périphérique diabétique selon la revendication 1 ou 2, dans laquelle 400 mg de la préparation de sang de veau déprotéiné sont administrés trois fois par jour par voie orale.

4. Utilisation d'une préparation de sang de veau déprotéiné pour la préparation d'un médicament à utiliser dans la prévention ou le traitement de la polyneuropathie périphérique diabétique selon l'une quelconque des revendications 1 à 3, dans laquelle 600 mg de la préparation de sang de veau déprotéiné sont administrés trois fois par jour par voie orale.

5. Utilisation d'une préparation de sang de veau déprotéiné pour la préparation d'un médicament à utiliser dans la prévention ou le traitement de la polyneuropathie périphérique diabétique selon l'une quelconque des revendications 1 à 4, dans laquelle la préparation de sang de veau déprotéiné est administrée trois fois par jour par voie orale pendant 140 jours.

6. Utilisation d'une préparation de sang de veau déprotéiné pour la préparation d'un médicament à utiliser dans la prévention ou le traitement de la polyneuropathie périphérique diabétique selon l'une quelconque des revendications 1 à 5, dans laquelle la préparation de sang de veau déprotéiné est administrée trois fois par jour par voie orale pendant 150 jours voire même plus longtemps en tant que traitement sensiblement continu.

7. Préparation de sang de veau déprotéiné pour utilisation dans la prévention ou le traitement de la polyneuropathie périphérique diabétique, dans laquelle 1000 mg ou 2000 mg de la préparation de sang de veau déprotéiné sont administrés quotidiennement par voie intraveineuse pendant une période d'au moins 10 jours consécutifs, et dans laquelle l'administration par voie intraveineuse est suivie par une période d'administration par voie orale de 200 mg, de 400 mg ou de 600 mg de la préparation de sang de veau déprotéiné en une, deux ou trois prises quotidiennes pendant une période de 120 jours voire même plus longtemps en tant que traitement sensiblement continu.

8. Préparation de sang de veau déprotéiné pour utilisation selon la revendication 7, dans laquelle 250 ml de la préparation de sang de veau déprotéiné ayant une teneur en substances actives de 8 mg/ml est administrée une fois par jour par voie intraveineuse.

9. Préparation de sang de veau déprotéiné pour utilisation selon la revendication 7 ou 8, dans laquelle 400 mg de la préparation de sang de veau déprotéiné sont administrés trois fois par jour par voie orale.

10. Préparation de sang de veau déprotéiné pour utilisation selon l'une quelconque des revendications 7 à 9, dans laquelle 600 mg de la préparation de sang de veau déprotéiné sont administrés trois fois par jour par voie orale.

11. Préparation de sang de veau déprotéiné pour utilisation selon l'une quelconque des revendications 7 à 10, dans laquelle la préparation de sang de veau déprotéiné est administrée trois fois par jour par voie orale pendant 140 jours.

12. Préparation de sang de veau déprotéiné pour utilisation selon l'une quelconque des revendications 7 à 11, dans laquelle la préparation de sang de veau déprotéiné est administrée trois fois par jour par voie orale pendant 150 jours voire même plus longtemps en tant que traitement sensiblement continu.
